# EUROPEAN PATENT APPLICATION

(11) **EP 1 953 237 A1**
(43) Date of publication of application: **06.08.2008**
(21) Application number: 07101581.2
(22) Date of filing: 01.02.2007
(51) Int. Cl.: C12P 19/44, C07H 15/04, C11D 3/22

(54) **A method for the production of short chained glycolipids**

(71) Applicant: ECOVER N.V., Curaçao (AN)
(72) Inventor: Develter Dirk, 9990, Maldegem (BE); Fleurackers, Steve, 2100, Deurne (BE)
(74) Representative: Luys, Marie-José A.H.

(57) **Abstract**

This invention relates to a method for producing a glycolopid wherein at least one micro-organism strain capable of producing glycolipids is contacted with at least one carbohydrate substrate and at least one hydrophobic hydrocarbon substrate which responds to the chemical formula I or a salt or a methyl, ethyl or glycerol ester of the compound of formula I which contains at least one cleavable bond,

R¹⁰-R¹-X-Y-Z-R²-R²⁰ (formula I)

wherein
- Y is -O-, -S-, -NH-, a mono or di unsaturated bond
- X and Z may be the same or different and are chosen from the group of a-CO- group or a -CHₙ(CH₂)ₘ- group with m having a value between 0 and 4 and n between 0 and 2 which may be straight or branched
- R¹ and R² each are aliphatic hydrocarbon chains which may be the same or different chain, which may be branched or unbranched, may contain one of more unsaturated bonds and may contain one or more substituents
- R¹⁰ and R²⁰ may be the same or different and are chosen from the group of
-H, -CH₃, -CHO, -CH₂OH, -COOH, -CHS, -CH₂SH, -CN or -CH₂NR³ₚ, R³ being an aliphatic hydrocarbon chain or H, p being 1 or 2
- -R¹⁰-R¹-X is a lipophilic moiety of the glycolipid
- -Z-R²-R ²⁰ is an elongator part of the compound of formula I

wherein the number of atoms in the chain determining the chain length of formula I is between 14 and 22, preferably between 16 and 18
and in that the at least one cleavable bond is broken to remove the elongator part from the sophorolipid to obtain a short chained glycolipid.

## Description

The present invention relates to a method for method for producing a glycolopid wherein at least one micro-organism strain capable of producing glycolipids or an acetylated derivative thereof when contacted with a carbohydrate and a hydrophobic aliphatic substrate, is contacted with at least one carbohydrate substrate and at least one hydrophobic aliphatic substrate in aqueous medium which is to be converted into at least one glycolipid, according to the preamble of the first claim. The present invention relates in particular to a method for producing short chain sophorolipids.

### Prior art.

The use of glycolipid biosurfactants in detergents is well known in the art. Glycolipid biosurfactants include rhamnolipids, sophorolipids, cellobioselipids, trehaloselipids, mannosyl erythritol lipids and (bio)chemical modifications thereof. Glycolipid based biosurfactants are understood to comprise those surfactants that have been obtained through microbial cultivation and consist of carbohydrates bound to aliphatic acids or aliphatic hydroxyl acids through glycosidation or acylation. They offer the advantage of being naturally produced molecules that can be produced through microbial cultivation by feeding renewable raw materials and of being fully degradable after use.

Sophorolipids are one of the most promising glycolipids known, one reason being their high production yield and ease of recovery from the microbial cultivation. Several Candida species, a.o. *Candida bombicola* and *Candida apicola,* are known to produce sophorolipids in large amounts from various substrates such as carbohydrates, vegetable oils, animal fats and n-alkanes. Sophorolipid production mainly takes place during the stationary phase because of nitrogen limitation. It appears to be enhanced when providing simultaneously hydrophilic (e.g. glucose) and hydrophobic (e.g. fatty acids) substrate to Candida species. Candida bombicola for example produces a complex mixture of 22 structurally different sophorolipids from either glucose and or an oily substrate, preferably C16 to C18 alkanes, fatty acids or their esters. The main compounds produced are the lactonic and the acidic form typically in the ratios of 75 and 25 w/w. A typical hydroxyl fatty acid distribution incorporated in unmono- and diacetylated sophorolipids either in their free acid or lactonic form is for example disclosed in FR2779057:

**Table 1.**

| Type | Hydroxy fatty acid | Wt. % |
|---|---|---|
| C16:0 | 15-OH hexadecanoic | 1.5 |
| C16:0 | 16-OH hexadecanoic | 2 |
| C18:0 | 17-OH octadecanoic | 3.5 |
| C18:1 | 17-OH octadecenoic | 60 |
| C18:1 | 18-OH octadecenoic | 12 |
| C18:2 | 17-OH octadecadienoic | 7 |
| C18:2 | 18-OH octadecadienoic | 14 |

Sophorolipids find numerous applications for example in preventing and curing dandruff and body odor attributed to bacteriostatic properties as disclosed by EP-A-1.082.097, as therapeutically active substances or cosmetic products, in particular skin treatment as disclosed by EP-A-835.118, for their antifungal properties disclosed by US-A-2005/0164955. EP-A-820.273 discloses that sophorolipids are non-irritant to the skin, weakly irritant to the eyes only and that they show anti-inflammatory as well as elastase inhibiting activity. Other uses of sophorolipids include their use as the sole surfactant in washing and cleaning applications such as disclosed in US-B1-6.433.152. According to EP-A-1.445.302 mixtures of sophorolipids show synergistic activity in laundry and hard surface cleaning applications. EP-A-499.434 discloses to combine sophorolipids with lamellar, usually ethoxylated non-ionic surfactants in washing and cleaning applications. DE-A-19600743 discloses manual dishwashing formulations based on sophorolipids and other glycolipids combined with high foaming surfactants.

However, the physicochemical properties of sophorolipids form an important limitation to their applicability in the sense that the lactonic form of the sophorolipids is hardly water soluble, it is insoluble in acidic environment, it is spontaneously deacetylated in alkaline pH and upon deacetylation the pH reduces to approximately 6. Moreover fully deacetylated acidic sophorolipids lose a substantial part of their surface activity with respect to the diacetylated lactonic form of crude sophorolipids. Several attempts have been made to produce shorter-chained sophorolipids, as those are presumed to decrease surface tension more effectively than the known long chain C16-C22 sophorolipids. *Candida bombicola* is capable of readily fermenting C16 and C18 chains and of incorporating them into the glycolipids. However shorter hydrocarbon chain precursors C10-C14 were found not to be incorporated by the micro-organism, but assimilated instead. C15-sophorolipids were obtained in unsatisfactory yields of below 40 % by Jones and Howe (1968), by offering a wide variety of expensive C15 substrates to *Torulopsis gropengiesseri.* The terminal methyl group of methyl esters is not incorporated in the end product, so basically it does not make any difference whether a carboxylic acid or its esters are used.

Traces of a C14 component could only be detected when a glucose/tetradecane mixture was used as hydrocarbon source. According to the teaching of US-B-6433152 short chain 3-alkanols, 4-alkanols and 2, 3 and 4-alkanols could be converted into short chain sophorolipids with the envisaged chain length, provided the conversion was carried out under reduced oxygen concentrations and with an exceptionally high glucose feed. However, the yield was only between 3-17 g/l, of which only 41-85 % had the envisaged short chain length. Although US-B-6433152 claims incorporation of the easily obtainable and cost-effective 1-alcohols, no examples are given or yields specified. It therefore seems like to date is has not been possible to use 1-alcohols for the production of short-chain sophorolipids, as these are first converted into the corresponding acids before they are transformed into glycolipids rather than being glycosidically bound at the hydroxyl group (Jones et al, 1968). According to the disclosure of DE19518982.5 *Candida* species are capable of converting secondary alcohols or ketones to non-cyclic sophorose lipids with surface active properties. The yields amount to approximately 20 g/l of C12-C14 sophorolipids, 75-85 % of which has the desired chain length. 7% was converted into a fatty diol. However, the low yield, combined with the impurity of the end product which is a mixture containing 10% of standard C18 sophorolipids and the fact that secondary alcohols are expensive, render this process unattractive.

In most *Candida* species, substrates of more than 18 carbon atoms are mainly reduced in chain length by one or more two-carbon units and hydroxylated, thus yielding C17 or C18 hydroxy fatty acids with the OH group on the penultimate carbon atom (Tulloch, Spencer and Gorin 1962). Depending on the degree of unsaturation and the hydrocarbon chain length, hydroxylation will either occur on the terminal or the penultimate carbon atom. This enzymatic hydroxylation appears to be fairly specific in its action, since the relative amounts of hydroxylation at the w or ω-1 position can be correlated with the length of the substrate molecule. A double bond for example spatially shortens the fatty acid chain length, causing oleic acid to be w and ω-1 hydroxylated, whereas stearic acid apparently is too long for ω-hydroxylation and is therefore hydroxylated only at the ω-1 position. Pentadecanoic acid proved to be too short for appreciable hydroxylation and 12-hydroxyoctadecanoic acid did not react because the hydroxyl group is too close to the reaction site. On the other hand, substituents at the 8- and 10-position appear to be too far away to interfere.

### Description of the invention.

There is thus a need for a process with which short-chain glycolipids or an acetylated derivative thereof, in particular sophorolipids or an acetylated derivative thereof, more in particular sophorolipids with a hydrocarbon chain length of C15 or less, can be produced at an acceptable yield,
wherein the process shows a sufficient selectivity towards the desired sophorolipid.

It is therefore the object of the present invention to provide a process for producing short-chain glycolipids, preferably sophorolipids, in particular sophorolipids with a hydrocarbon chain length of 15 or less, at an acceptable yield, and a selectivity towards the desired sophorolipid which is sufficiently high.

This is achieved according to the present invention with the technical features of the characterizing part of the first claim.

Thereto the method of the present invention is characterized in that as the hydrophobic hydrocarbon substrate use is made of a compound which responds to the chemical formula I or a salt or a methyl, ethyl or glycerol ester of the compound of formula I which contains at least one cleavable bond,

R¹⁰-R¹- X-Y-Z-R²-R²⁰ (formula I)

wherein
- Y is -O-, -S-, -NH-, a mono or di unsaturated bond
- X and Z may be the same or different and are chosen from the group of a - CO- group or a -CHₙ(CH₂)ₘ- group which may be straight or branched, with m having a value between 0 and 4 and n between 0 and 2
- R¹ and R² each are aliphatic hydrocarbon chains which may be the same or different chain, which may be branched or unbranched, may contain one of more unsaturated bonds and may contain one or more substituents
- R¹⁰ and R²⁰ may be the same or different and are chosen from the group of
- H, -CH₃, -CHO, -CH₂OH, -COOH, -CHS, -CH₂SH, -CN or -CH₂NR³ₚ, R³ being an aliphatic hydrocarbon chain or H, p being 1 or 2
- -R¹⁰-R¹-X is a lipophilic moiety of the glycolipid
- -Z-R²-R²⁰ is an elongator part of the compound of formula I
wherein the number of atoms in the chain determining the chain length of formula I is between 14 and 22, preferably between 16 and 18 and in that the at least one cleavable bond is broken to remove the elongator part from the glycolipid to obtain a short chained glycolipid.

Depending on the nature of X-Y-Z, Y may remain connected to the ultimate lipophilic moiety of the glycolipid, for example in case -X-Y-Z is an ester or an amide, or to the elongator part or may be completely removed. Complete removal will for example occur in case -X-Y-Z- is a -CH=CH- or a -C=C- moiety. An examples where Y remains bound to the glycolipid include the situation where X= -CH₂-, Y= -NH- and Z = -CO-. After hydrolysis, N is contained in the terminal group of the glycolipid. In case X and Z are exchanged, the carboxylic group remains attached to the glycolipid and the N containing part is removed with the elongator. Another example includes the situation where X = - CO-, Y = O and Z = -CH₂-. Upon hydrolysis of the ester a glycolipid is formed with a terminal COOH. A terminal OH is formed in case X and Z are exchanged. Upon ozonolysis of a compound where X and Z are C and Y is a triple unsaturated bond, a glycolipid is formed, in which both X and Z are transformed into a carboxyl or aldehyde group.

In the method of this invention the micro-organism is contacted with the fatty substrate compound of formula I, which contains at least one cleavable bond. The cleavable bond may either be an unsaturated carbon-carbon bond or a hetero-atom - carbon bond. As a result, a glycolipid or or an acetylated derivative thereof is released by the micro-organism. Within the scope of the present application glycolipid refers to the glycolipid as such as well as to its acetylated derivative.

In a preferred embodiment, the compound of formula I is a so called shielded ester, wherein the hydrocarbon chain which determines the chain length of each of R¹⁰-R¹-X- and -Y-Z-R²-R²⁰ contains at least 3 carbon atoms. The R¹⁰-R¹- and/or -R²-R²⁰ part of the shielded ester may contain unsaturated carbon-carbon bonds. Within the scope of the present invention, a shielded ester designates a compound represented by formula I, wherein the functional group -X-Y-Z- is spaced apart from the end part of formula I by 3 or more carbon atoms.

Such shielded, cleavable esters may be synthesised chemically or enzymatically using methods well known to the person skilled in the art. In this shielded ester the functional group -X-Y-Z- is sufficiently far away from the terminal part of the molecule, so that its accessibility to extra-cellular enzymes is minimal. Although the use of conventional terminal esters - i.e. methyl-, ethylesters and glycerides - as substrates for sophorolipid synthesis has been well documented, these esters are readily hydrolysed by extracellular enzymes into C16-18 carboxylic acids. Terminal esters will mainly yield C18 sophorolipids, which can be converted into shorter chained sophorolipids in case they contain one or more unsaturated carbon-carbon bonds. According to the present invention this is achieved by subjecting the C18 sophorolipid to ozonolysis as is described below.

The inventors have now found that regardless of the presence of a functional group in the chain which determines the chain length of the compound of formula I, and regardless of the fact that the functional group may contain hetero atoms that contribute to the chain length such as for example O, N, S, the compound of formula I is incorporated by the micro-organism into the corresponding glycolipid in the usual way, similarly to the way in which a conventional carboxyl or terminal ester substrate which does not contain such hetero atoms in the chain length would be incorporated. It has been found that once absorbed by the micro-organism, the compound of formula I is subjected to the usual two-step hydroxylation and glycosidation by glycosyltransferase to yield the corresponding glycolipid, which glycolipid contains the compound of formula I. In practice this means that the compound of formula I is incorporated into the glycolipid with -R¹⁰-R¹-X bound to the saccharide part of the glycolipid. This is unexpected. Apparently, the shielded ester is seen by the micro-organism as a molecule with a hydrocarbon chain with 16-18 carbon atoms, although the shielded ester contains in the part that determines its chain length, at least one hetero-atom. For example, in a shielded ester containing a fatty alcohol which is esterified with a dicarboxylic acid to an apparent C16-18 ester, in the carboxylic group the oxygen atom bound to the C=O moiety is seen by the micro-organism as an apparent carbon atom.

To obtain the short chained glycolipid, the elongator part -Y-Z-R²-R²⁰ is removed from the glycolipid or its acetylated form that is released by the micro-organism, by subjecting the glycolipid released by the micro-organism to a process which cleaves the cleavable bond, but which leaves the connection of the R¹-R¹⁰- moiety to the saccharide part of the glycolipid unaffected. By properly selecting the nature and chain length of the compound providing the lipophilic part to be incorporated into the glycolipid and the elongator part which is to be removed, glycolipids of varying chain lengths and varying nature may be produced.

The preferred saccharide is glucose, the preferred glycolipid is a sophorolipid. However, the technology of this invention can also be applied to glycolipids containing another saccharide part. Glycolipids suitable for use within the framework of this invention include those containing a sophorose, rhamnose, cellobiose, trehalose, glucose or oligoglucose carbohydrate moiety as the saccharide part. This invention therefore includes for example unsaturated rhamnolipids synthesised by Candida Bombicola, which today do not exist as well as any (potentially undescribed) native glycolipids that are either unsaturated or incorporate the shielded ester of Formula I.

According to a preferred embodiment in the -CH₂NR³ₚ moiety R³ is H or a CₓH₂ₓ₋₁ group, wherein x preferably varies from 1-12. Preferred - CH₂NR³ₚ moieties include -NH₂, -N(CH₃)₂, -N(C₂H₅)₂.

A preferred embodiment of the present invention is characterized in that in formula I, the hydrocarbon chain which determines the chain length of each of R¹⁰-R¹-X and -Y-Z-R²-R²⁰ contains at least 3 carbon atoms. Such a compound is called a "shielded ester". In the shielded ester of formula I, the functional group -X-Y-Z- containing the cleavable bond, is situated in an inner part of the structural formula and is thus not a terminal group. The shielding of the -X-Y-Z- functional group on both sides by hydrophobic hydrocarbon chains has been found to inhibit access of extracellular enzymes to it. Thus, the cleavable bond is protected against the action of the extra-cellular enzymes, a.o. against hydrolysis. As a result, the selectivity towards the desired glycolipid is increased.

Preferably, the compound of formula I is a directional shielded ester, in which R¹⁰ is -CH₃ as a hydrophobic terminal group on the end part of formula I which is to be connected to the saccharide part of the glycolipid. R²⁰ is the hydrophilic terminal part on the opposite side of the compound, preferably a -CH₂OH, -CH₂SH, -CH₂NH₂, or -COOH group. Such compounds may be obtained from compounds in which the elongator part -Y-Z-R²-R²⁰ is derived from compounds of the group of a dioic acid, a diol, an amino acid or a short chain hydroxyl fatty acid, and in which the elongator part is bound to a lipophilic moiety which is selected from the group of a fatty acid, a fatty amine, a fatty alcohol. The presence of this polar group forces the micro-organism to hydroxylate the opposite side and to bind to the R¹⁰ moiety, thus improving the selectivity of the reaction and reducing the number of glycolipid side products. Using this substrate, hydroxylation during synthesis of the glycolipid mainly takes place at the ultimate or penultimate carbon of the non-polar or lipophilic side of the molecule.

Thus, the use of the shielded ester having a hydrophilic terminal group at one end, controls the course of the reaction and drives the reaction in such a way that hydroxylation by the micro-organism takes place at the other end i.e. at the lipophilic part R¹⁰-R¹-X, which preferably is a short chained fatty alcohol part of the ester. As a result, a higher selectivity towards the wanted glycolipid can be achieved. The use of a non-directional shielded ester with a lipophilic terminal group rather than the hydrophilic terminus provided by the elongator part leads to mixtures of glycolipids with hydroxylation taking place at either one of the two termini as stated in formula I, as is observed when starting from esters made out of mono-alcohols and mono-carboxylic acids. The result is a lower selectivity towards the desired glycolipid.

In a further preferred embodiment -X-Y-Z- is an ester as this provides a cleavable bond which is readily hydrolysable in alkaline medium, while at the same time leaving the connection of R¹ to the saccharide part of the glycolipid unaffected.

The person skilled in the art will be capable of adapting the nature of R¹⁰-R¹-X- and the nature of the elongator part -Z-R²-R²⁰ of formula I in such a way that a shielded ester with an apparent chain length of 16-18 atoms is obtained. The nature of the compound providing the hydrophilic elongator part may be varied within wide ranges and may for example be a dicarboxylic acid, for example aspartic or glutamic acid which may be obtained in their L-form upon fermentation. Alternatively, short chain hydroxyl fatty acids such as lactic acid could be used, or diols such as 1, 3-propanediol. Thus a C12-14 fatty acid as the lipophilic part can be esterified to a fully vegetable carbon source with an apparent chain length of C16-18. Alternatively cheap and readily available C12-14 fatty alcohol can be esterified with malonic acid as the hydrophilic part or a C13-15 fatty acid can be esterified with glycolic acid as the hydrophilic part to obtain an apparent C16-18 ester with a terminal carboxyl group. In the latter case the fatty alcohol forms the lipophilic part; the glycolic acid moiety forms the elongator part.

Preferably the sum of the number of atoms in the part of the lipophilic part R¹⁰-R¹-X and - the elongator part Z-R²-R²⁰ which determines the chain length thereof, is between 14 and 19, preferably between 15 and 18 as this provides a substrate with an apparent chain length of between 15 and 20, preferably between 16 and 19 which is most readily fermented by the micro-organism.

Within the scope of the present invention the lipophilic part R¹⁰-R¹-X- of the compound of formula I which is built into the glycolipid, is preferably an aliphatic moiety which may be a straight chain or branched, saturated or unsaturated aliphatic moiety, and which may contain one or more functional groups for example an OH or NH₂ group; an alcohol, diol or polyol moiety a carbonyl or carboxylic acid moiety, which may be a straight chain or branched, saturated or unsaturated, containing between 3 and 15 carbon atoms.

The -Z-R²-R²⁰ moiety of the compound of formula I is preferably an aliphatic moiety which may be a straight chain or branched, a saturated aliphatic moiety or an aliphatic moiety containing one or more unsaturated bonds, and contain one or more functional groups for example an OH or NH₂ group, an alcohol, diol or polyol moiety; a carbonyl or carboxylic moiety, which may be a straight chain or branched, saturated or unsaturated, containing between 3 and 15 carbon atoms. More preferably, the elongator part is a dithiol, a diol, a dicarboxylic acid, an amino acid or a hydroxyl fatty acid containing between 3 and 15 carbon atoms, as such compounds can be more easily synthesized and the elongator part can be more easily removed. Most preferably -R²-R²⁰ is derived from a compound selected from the group of short chained dioic acids, diols or hydroxy fatty acids containing between 3 and 14 carbon atoms.

Preferably this elongator part is esterified with a fatty moiety R¹⁰-R¹- selected from the group of a fatty acid, a fatty amine, a fatty alcohol. Suitable examples of dioic acids include pimelic acid, suberic acid, azlaic acid, adipic acid, glutaric acid, malonic acid, succinic acid, oxalic acid. These are preferred as they are readily commercially available.

Suitable diols for use in the present invention include 1,4- butadiol, 1-3 propadiol. These are preferred as they are readily commercially available. Suitable examples of the elongator part also include aspartic or glutamic acid and the like, which may for example be obtained in their L-form by fermentation. Alternatively short chain hydroxyl fatty acids such as lactic acid or diols, such as 1,3-propanediol could be used and ester-bound to the desired carboxylate. Thus a C₁₂₋₁₄ fatty acid can be esterified to a fully vegetable carbon source with an apparent chain length of C₁₆₋₁₈. Alternatively a cheap C₁₂₋₁₄ fatty alcohol could be esterified with malonic acid or a C₁₃₋₁₅ fatty acid could be esterified with glycolic acid to obtain apparent C₁₆₋₁₈ esters with a carboxylic terminal group. The selection of the most appropriate elongator part and lipophilic part will usually be determined by economic parameters, such as its commercial availability and price.

The compound of formula I preferably is a fatty alcohol which is esterified with a dioic acid.

Examples of shielded esters suitable for use in the present invention are given in table 2 below. Other suitable substrates for use in the present invention include unsaturated fatty moieties, for example oleic acid and linoleic acid, or their terminal esters.

**Table 2.**

| | | | | |
|---|---|---|---|---|
| H-(CH₂),-O-CO-(CH₂),-COOH | | | | |

| **n** | **monoAlcohol** | **m** | **diAcid** | **Total** |
|---|---|---|---|---|
| 8 | 1-octanol | 5 | Pimelic | 16 |
| 8 | 1-octanol | 6 | Suberic | 17 |
| 8 | 1-octanol | 7 | Azelaic | 18 |
| 9 | 1-nonanol | 4 | Adipic | 16 |
| 9 | 1-nonanol | 5 | Pimelic | 17 |
| 9 | 1-nonanol | 6 | Suberic | 18 |
| 10 | 1-decanol | 3 | Glutaric | 16 |
| 10 | 1-decanol | 4 | Adipic | 17 |
| 10 | 1-decanol | 5 | Pimelic | 18 |
| 11 | 1-undecanol | 2 | Succinic | 16 |
| 11 | 1-undecanol | 3 | Glutaric | 17 |
| 11 | 1-undecanol | 4 | Adipic | 18 |
| 12 | 1-dodecanol | 1 | Malonic | 16 |
| 12 | 1-dodecanol | 2 | Succinic | 17 |
| 12 | 1-dodecanol | 3 | Glutaric | 18 |
| 13 | 1-tridecanol | 0 | Oxalic | 16 |
| 13 | 1-tridecanol | 1 | Malonic | 17 |
| 13 | 1-tridecanol | 2 | Succinic | 18 |
| 14 | 1-tetradecanol | 0 | Oxalic | 17 |
| 14 | 1-tetradecanol | 1 | Malonic | 18 |
| 15 | 1-pentadecanol | 0 | Oxalic | 18 |

| | | | | |
|---|---|---|---|---|
| * the total length of the resultant ester as compared to a normal, linear fatty acid | | | | |

n is the number of carbon atoms in the chain length determining part of the alcohol and m the number or carbon atoms in the chain length determining part of the carboxylic acid minus two.

Removal of the elongator part from the glycolipid or its acetylated derivative obtained using the above disclosed shielded ester, can be done using any technique considered suitable by the person skilled in the art. However, care has to be taken to select a technique which not only leaves the lipophilic part attached to the glycoside unit of the glycolipid, but which also leaves this lipophilic part unaffected. Therefore, the cleavable bond is preferably contained in a moiety which is selected from the group of a carboxyl group, an ester, an amide group, an anhydride group, a S - C=O group and the reaction product delivered by the micro-organism is subjected to hydrolysis in alkaline reaction medium to hydrolyse the cleavable bond and to remove the elongator part -Z-R²-R²⁰. Alkaline hydrolysis is particularly suitable in case Y is O or NH and -Z-R²-R ²⁰ is a dithiol, a diol, diacid, amino acid or hydroxy fatty acid moiety.

The result of the hydrolysis is a glycolipid with the desired chain length, containing a hydroxyl group, an amine, a carboxyl group, an SH group as a terminal group, depending on the nature of the cleavable bond. Alkaline hydrolysis also removes all acetate groups present on the glycolipid.

The alkaline hydrolysis is preferably carried out at a pH which is at least 8, preferably at least 11, more preferably at least 13. To obtain this pH, use can be made of any strong base considered suitable by the person skilled in the art. Preferably use will be made of a base of an alkaline metal, for example NaOH or KOH. The alkaline hydrolysis can be carried out at a temperature in the vicinity of room temperature. However, to increase the reaction rate the alkaline hydrolysis may also be carried out at elevated temperature, for example at reflux temperature or any temperature between room temperature and reflux temperature. The temperature is however chosen such that there is a minimum risk to decomposition of the glycolipid.

Within the framework of this invention, the cleavable bond may be any bond which is readily hydrolysable in alkaline medium, as this leaves the bond between the glycosyl unit and the hydrophilic part unaffected. Suitable examples of compounds for use to provide the lipophilic part -R¹⁰-R¹-X include a fatty acid, a fatty amine or a fatty alcohol. Suitable compounds for use as the elongator part -Z-R²-R²⁰ esterified with the lipophilic part -R¹⁰-R¹-X include a dioic acid, a diol, an amino acid or a short chain hydroxy fatty acid. Preferably use is made of a fatty alcohol as -R¹⁰-R¹-X which is esterified with a dioic acid as -Y-Z-R²-R²⁰, as this provides a -O-CO- bond which is readily hydrolysable in alkaline medium. For example: dodecanol and glutaric acid may be esterified to form R¹⁰ = CH₃-; R¹= (CH₂)₁₀-; X = CH₂-; Y = O-; Z = CO-; R² = -(CH₂)₃; R²⁰ = -COOH. In that way for example 1-decanol can be esterified with glutaric, adipic or pimelic acid to obtain a carboxyl terminated shielded ester with an apparent chain length of respectively 16, 17 or 18 which are all suitable for fermentation by Candida bombicola. Those shielded esters are incorporated as such in high yield in the glycolipid produced by the micro-organism. After alkaline hydrolysis of the thus obtained glycolipid including the shielded ester, a short chain glycolipid results with a terminal hydroxyl group, which is less sensitive to water hardness than its carboxylic equivalent and is therefore preferred for cleaning applications. Within the scope of the present invention alkaline hydrolysis may be carried out in such a way that complete hydrolysis of the product containing the cleavable bond is obtained. Alkaline hydrolysis may however also be carried out in such a way that only part of the product containing the cleavable bond is hydrolysed.

Another suitable technique for cleaving the cleavable bond and obtaining short chained glycolipids, in particular in case Y corresponds to an unsaturated bond, i.e. in case -X-Y-Z- is a -C=C- or a -C=C- moiety, is ozonolysis. However, ozonolysis may also be carried out in addition to the alkaline hydrolysis, in case part of the obtained glycolipid mixture resulting from the alkaline hydrolysis contains at least one unsaturated C-C- bond. This permits shortening the lipophilic part of the glycolipid without affecting the glycosidic bonds and the carbohydrate part of the glycolipid, while maintaining the amphiphilic nature of the glycolipid. The inventors have also found that glycolipids produced by the micro-organism provide a broadened spectrum of products in the sense that besides incorporation of the product of formula I, also de novo synthesis of for example glycolipids with an unsaturated hydrocarbon chain takes place. The present invention now ensures that short chained glycolipids may not only be obtained from hydrocarbon chains containing a hydrolysable bond, but also from hydrocarbon chains containing one or more unsaturated bonds. When using an unsaturated substrate rather than a shielded ester the chain length of the lipophilic part of the glycolipid may be controlled by selecting the position of the unsaturated C-C bond. So, other lipophilic substrates suitable for use in the present invention include unsaturated fatty moieties or their terminal esters, the glycolipids of which are subjected to ozonolysis subject of this invention. For example the use of octadec-E6-acid (petroselenic acid) with Candida Bombicola will yield a C12 sophorolipid and a removed adipic acid part. Ozonolysis of standard monounsaturated C18 sophorolipid obtained from oleic acid will yield a C9 sophorolipid with a terminal carboxyl group.

Within the scope of the present invention ozonolysis may be carried out in such a way that either complete or only partial ozonolysis of the product containing the cleavable bond is obtained.

Ozonolysis is preferably carried out in aqueous medium, at a pH of between 1 and 9, preferably between 4 and 8, more preferably between 5 and 6 as in this region foaming is reduced to a minimum and a sufficient solubility of the ozone in the reaction medium is guaranteed. When using a pH below 4 the reaction rate seriously deteriorates because of the decreasing solubility of the sophorolipid which reduces the contact with the ozone/H₂O₂, at pH above 8 too much peroxide is decomposed for sufficient reaction with the ozonide.

Ozonolysis will usually be carried out by flowing ozone enriched oxygen through the reaction mixture. The inventors found that ozonolysis selectively splits the unsaturated bond of the compound of formula I and that the risk to affecting the sophorose unit or to cleavage of the β-D-aidosic linkage between the glycose units is minimal, contrary to the teaching of Wang and coworkers (1998). The ozonolysis reaction is preferably carried out at room temperature, but it may be carried out at higher temperatures as well. At higher temperature in particular the reaction rate between the ozonide and the hydrogen peroxide will increase, but on the other hand decomposition of hydrogen peroxide will be enhanced and reduce the solubility of the O₂/O₃ mixture.

With the method of this invention glycolipids may be produced with a shorter hydrophilic part than hitherto known. The inventors have now found that according to the present invention, a high selectivity is obtained towards cleaving of the unsaturated bond, thereby leaving the glycosidic bond unaffected. This way a novel method is provided for producing short chained glycolipids, the chain length depending on the position of the unsaturated bond with respect to the glycosidic bond, the terminal group depending on the reaction pH. The method of the present invention permits cleaving the double bond present in the sophorolipid, without affecting the sophorose unit, nor the glycosidic bond. Oxidative splitting of unsaturated bonds in unsaturated aliphatic hydrocarbon chains, with the purpose of shortening the lipophilic part of the molecule is known from the art and is for example disclosed in US-A-2.813.113, US-A-3.402.108, EP-A-1.074.540 and WO90/05007. The latter discloses ozonolytic cleaving of oleic acid into azelaic acid and pelargonic acid. Azelaic acid is a particularly useful building block in polymer synthesis. However, according to Wang and coworkers (1998), β-D aldosidic linkages between glucose units are destroyed when contacted with ozone. Thus according to Wang and coworkers (1998), when subjected to ozonolysis, a glucose unit would be split off from the glycolipid.

Ozonolysis of the unsaturated glycolipid according to the present invention disclosed above, results in a short chain glycolipid and the removed part, which usually is a dicarboxylic acid. The inventors have found that in the course of the ozonolysis, the removed fatty moiety is oxidized to a terminal carboxylic acid group due to the presence of hydrogen peroxide. Thus carboxylic acids may be produced with chain lengths which are not as such available in nature and which can only be produced using complicated production processes, at relatively low yields and low selectivities. For example, sophorolipids obtained after fermentation of oleic acid C₁₈H₃₄O₂ (or CH₃(CH₂)₇CH=CH(CH₂)₇COOH) will yield a sophorolipid with a C9 carboxylic acid fatty moiety bound to it (SL9:OOH) and azelaic acid. Azelaic acid has a pronounced bacteriostatic activity even in low concentrations against a multitude of micro-organisms. Moreover, it reduces the presence of free fatty acids on the skin, which renders it very effective against acne. When using the raw ozonolysed reaction mixture the limited water solubility of azelaic is improved by the short chained sophorolipid leading to an increased effectivity. Alternatively, sophorolipids obtained after fermentation of petroselenic acid (6-octadecenoic acid) will yield a sophorolipid with a C12 carboxylic acid fatty moiety bound to it (SL12:OOH) and adipic acid.

Within the scope of the present invention, a complete ozonolysis or either a partial ozonolysis of the compound of formula I or its salt, methylester, ethylester or glycerolester may be performed. Partial ozonolysis is preferred in case it is the intention of improving glycolipid stability or surfactancy properties. Similarly, within the scope of this invention alkaline hydrolysis may be continued until it is complete or a partial hydrolysis may be carried out. Further, a partially hydrolysed glycolipid may be subjected to a full or partial ozonolysis, in order to retain at least part of the residual acetate groups on the carbohydrate moiety.

The present invention also relates to a method for producing hydroxyl fatty acids from the short chained glycolipid obtained with the above described method. Thereto, the glycolipid obtained with the above-described process is subjected to an enzymatic de-glycosidation, by contacting the glycolipid with glycosidase. Thereby, the glycolipid is split into a hydroxy fatty acid and a glycosidic part, which can be recovered. Alternatively, acid hydrolysis using a mineral acid can be employed to release the hydroxy fatty acid.

If so desired, the reaction product obtained after cleaving the cleavable bond, or preferably the product obtained from the alkaline hydrolysis and/or the ozonolyse, may be subjected to a hydrolysis reaction in acidic medium to break the glycosidic bond between the glycosyl unit of the glycolipid and the lipophilic part R¹. This is of particular importance in case the lipophilic part is a fatty acid moiety, as hydrolysis will result in the release of a hydroxy fatty acid. As the glycosidic bond with the fatty acid is usually formed at the ω or ω-1 position of the shielded ester depending on the nature of the fatty acid, acid hydrolysis will give ω and/or ω-1 hydroxyl fatty acids. For example, palmitic acid will usually yield ω hydroxy fatty acids, whereas stearic acid yields ω-1 hydroxy fatty acids. This type of fatty acids hardly occurs in nature and can only be synthesized using complicated processes, which usually produce the hydroxyl fatty acid in rather low yields and too low selectivities. The inventors have surprisingly found that the ω-1 hydroxyl fatty acids produced that way contain an optically active carbon atom carrying the OH group and are obtained in high purity in a stereochemical S-configuration. By properly selecting the nature of the compound of formula I, the length of the carbon chain of the glycolipid may be varied. By subjecting the glycolipid to an acid hydrolysis tailor made hydroxyl fatty acids may be produced. The present invention therefore also relates to a method for producing hydroxyl fatty acids.

The acid hydrolysis will usually be carried out by contacting the glycolipid with an acid medium having a pH in the range of between 0.1 and 3, preferably between 0.1 and 1. Thereto, usually use will be made of a strong acid, preferably a strong mineral acid, for example HCl. Acid hydrolysis will usually be carried out at elevated temperature to achieve that the reaction proceeds at a sufficiently high reaction rate.

Within the scope of the present invention, the glycolipid contains a glycosil unit which is selected from the group of sophorose, trehalose, cellobiose, rhamnose or glucose. However, sophorose is preferred. A preferred embodiment of this invention therefore relates to a method for producing sophorolipids, in particular short chained sophorolipids. Therefore, the preferred carbohydrate used in the method of this invention is a saccharide, more preferably sophorose, trehalose, cellobiose, rhamnose or glucose.

Within the framework of the present invention, sophorolipids are the compounds which correspond to the formula III or IV below : Formula III: 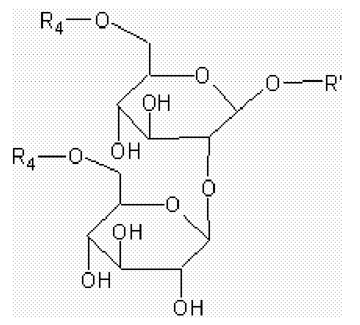 Herein, R⁴ is hydrogen or an acetyl group and R' is a - R¹⁰-R¹-X-Y-Z-R²-R²⁰ unit as described above. In the above the 6-ring members are 2-O-β-D-Glucopyranosyl-α-D-glucose. The sophorose group may be bound to R' on its ultimate or penultimate carbon atom. However, when using other micro-organisms the glycosyl unit may be trehalose, cellobiose, rhamnose, glucose and the like.

According to a preferred embodiment of the method of this invention, the glycolipid is a sophorolipid responding to the formula Gₙ-R¹⁰-R¹-X-Y-Gₘ in which G is a glucose unit bound to R¹⁰-R¹ on its ultimate or penultimate carbon atom and n and m may be the same or different and have a value of 0, 1 or 2.

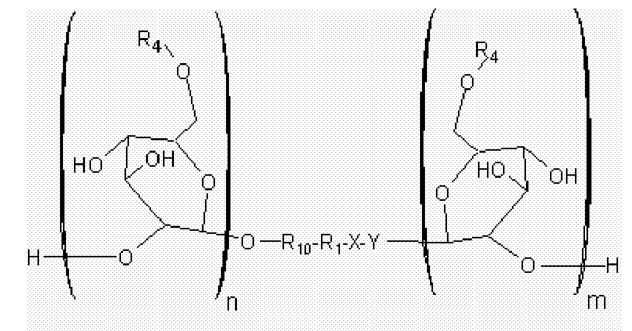

The micro-organism used in the method of the present invention may be any micro-organism considered suitable by the person skilled in the art, but is preferably selected from the group of as the micro-organism use is made of a micro-organism selected from the group of *Candida apicola, Candida bogoriensis, Candida Bombicola, Candida gropengiesseri, Candida magnoliae, Candida Antarctica Pseudozyma Antarctica), Candida tropicalis, Candida lipolytica, Wickerhamiella domercqiae.*

The present invention also relates to the sophorolipids as such, which respond to the formula 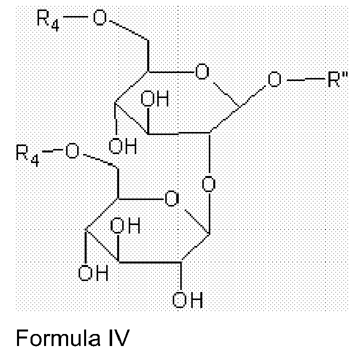 wherein
R⁴ is hydrogen or an acetyl group
R" is -R¹⁰-R¹-R³⁰ in which R³⁰ may be any of the following groups -CH₃, -CHO, - CH₂OH, -COOH, -CHS, -CH₂SH, -CH₂-NH₂, -CH₂NR³₂ (R³ is aliphatic hydrocarbon chain) or -CN. The sophorose group may be bound to R" on its ultimate or penultimate carbon atom.

The short chain glycolipids, in particular sophorolipids of the present invention can be used as such or can be converted into sophorose and hydroxyl fatty acids. When used as such they are suitable for use in domestic and industrial cleaning applications such as laundry washing, dishwashing, hard surface cleaning, in stay-on and rinse-off cosmetic applications. Examples of laundry compositions include heavy duty compact and regular laundry products, delicate wash, wool wash, specialty wash (for example for jeans or black garments), stain remover, stain sprays, fabric conditioners and laundry performance boosting additives. Examples of hard surface cleaning compositions include all purpose cleaners, cream cleanser, glass (window) cleaner, floor and wall cleaner, bathroom cleaner, mould remover, toilet cleaner, kitchen cleaner, oven cleaner, ceramic how and microwave oven cleaner and polishes.

Detergent compositions containing the glycolipids of the present invention can be used in any suitable form including powders, bars, tablets, liquids, sprays, pastes etc. Liquid compositions may be aqueous or nonaqueous. The glycolipids obtainable with the method of this invention may be used as such or in combination with other surfactants. Thus they can be combined with native glycolipids or non-glycolipid surfactants.

The present invention also relates to the use of the reaction mixture obtainable with the method of any one of claims 6-15 as an antimicrobial agent. In particular, the present invention relates to the use of the crude (unpurified) ozonolysed or hydrolysed reaction mixture obtainable with the above disclosed method for its antimicrobial properties, as the glycolipid has been found to improve the solubility of the elongator part, thus forming a synergistic mixture with improved antimicrobial effects.

The invention is further elucidated in the appending examples.

The following esters were synthesised using analytical grade chemicals (except for T13 where technical grade laurylalcohol was used) according to methods well known in the art, and used in the *Candida bombicola* fermentations described below. Any residual solvent was removed. HPLC-ELSD RP18 was used to verify the completeness of the reaction and the purity of the ester.

**Table 3.**

| **name** | **ester** | **Chemical structure** | **Number of C atoms bound to sophorose** | **Directional ester?** |
|---|---|---|---|---|
| T2 | Dodecyl | CH₃-(CH₂)₁₁-O-CO-(CH₂)₃- | 12/5 | No |
| | pentanoate | CH₃ | | |
| T5 | Pentenyl | CH₃-(CH₂)₄-O-CO-(CH₂)₁₀- | 12/5 | No |
| | dodecanoate | CH₃ | | |
| T10 | Decyl | CH₃-(CH₂)₉-O-CO-(CH₂)₅- | 10/7 | No |
| | heptanoate | CH₃ | | |
| T13 | Lauryl malonate | CH₃-(CH₂)_{11/13}-O-CO-CH₂- | 12/14 | Yes |
| | | COOH | | |
| T0 | Dodecyl | CH₃-(CH₂)₁₁-O-CO-(CH₂)₃- | 12/5 | Yes |
| | glutarate | COOH | | |

| Ester | R¹⁰ | R¹ | x | Y | Z | R² | R²⁰ |
|---|---|---|---|---|---|---|---|
| T2 | CH₃- | -(CH₂)₁₀- | -CH₂- | O | -CO- | -(CH₂)₃- | -CH₃ |
| T5 | CH₃- | -(CH₂)₃- | -CH₂- | O | -CO- | -(CH₂)₁₀- | -CH₃ |
| T10 | CH₃- | -(CH₂)₈- | -CH₂- | O | -CO- | -(CH₂)₅- | -CH₃ |
| T13 | CH₃- | -(CH₂)_{10/12}- | -CH₂- | O | -CO- | -CH₂- | -COOH |
| T0 | CH₃- | -(CH₂)₁₀- | -CH₂- | O | -CO- | -(CH₂)₃- | -COOH |

### Example E.1. Shake flask fermentation of dodecyl glutarate, ester T0.

A one litre baffled shake flask was filled with 200 ml medium according to Lang and co-workers (2000) and inoculated with Candida bombicola and left to grow at 30°C and 200 rpm. After 48 h 10 g/l Dodecyl glutarate was added. On day 5, 6 and 7 another 7.5 , 5 and 7.5 g/l ester was added. On day 10 the fermentation was stopped. The number of colony forming units was somewhat lower compared to fermentations on rapeseed oil or glucose. Moreover it dropped from approx. 4 to 1.10⁹/ml at the end of the experiment, suggesting a moderate toxicity effect of the ester. The sophorolipid yield as gravimetrically determined amounted 26 g/l. The residual ester concentration was 13 g/l. Dodecyl glutarate is a solid which was difficult to dose and tended to agglomerate, thus limiting the availability to Candida and probably causing part of the residual substrate. A HPLC analysis clearly revealed two new peaks, which are more hydrophilic than de novo synthesised sophorolipids which were observed as well (at approx.30 min retention time). These peaks were identified by RP-HPLC-MS (Waters ZQ) using quadrupole electrospray negative ion detection (see figure 1). They were shown to correspond to sophorolipids with the hydroxylated ester substrate as fatty moiety (hydroxy dodecyl glutarate). Both the lactonic and the acid diacetylated form were identified as well as traces of monoacetylated species.

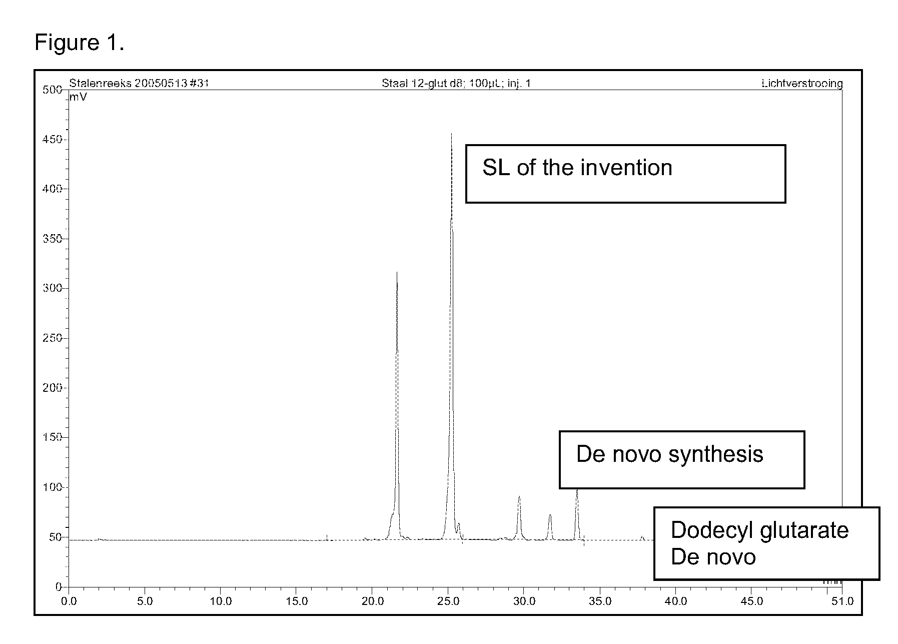

### Example E.2. Screening for toxic effects of glutaric acid.

To verify whether the ester or the glutaric acid which may be released following hydrolysis during the fermentation were toxic, 200 ml cultures were set up in one litre shaking flasks at 200 rpm. After 48 hours glutaric acid was added; 0, 10 or 100 %, the latter corresponding to the maximum amount of glutaric acid after hydrolysis of 30 g/l dodecyl glutarate (which equals the total amount of substrate added for synthesis). Eight days after the initial addition of glutaric acid the fermentation was stopped. The glutaric acid did not inhibit growth in terms of colony forming units or CFU nor did it lower the sophorolipid production by *Candida bombicola.* It is therefore concluded that the toxicity of dodecyl glutarate is due to the product itself, rather than to hydrolysis products.

### Example E.3. Dodecyl glutarate fermentation in fermentor vessel.

Taking into account the above results, a new experiment was set up in a Biostat B5 containing 3 litre medium to which 4g/l rapeseed oil was added in order to avoid toxic effects during the growth phase. From 48 hours onwards small portions of dodecyl glutarate, ester T0 in table 3 were added daily, totalling 145.5 gram during 7 days. Dodecyl glutarate has the same apparent chain length as stearic acid. After the last addition on day 8, the fermentation was run for another 48 hour and terminated 240 hours after the start. The fermentor was operated at 800 rpm mixing speed, 30°C and 1 vvm aeration, always assuring far higher oxygen concentrations than the 15% or less described in US6433152. After 140 hours the initial glucose was almost completely consumed and an additional 43 g/l was added. The pH was kept at 3.5 with 5 M NaOH and the oxygen saturation was varied between 70 and 85% during the production phase, rising 10 to 20 hours after each addition, which indicated the substrate was more or less consumed. The number of CFU in the production phase remained at approximately 6.10⁹/ml, the dry cell weight at about 15 g/l, both values being considerably lower than typical values for rapeseed oil fermentations with resp. 10¹⁰ CFU/ml and 25 g/l, again indicating moderate toxicity. Microscopical inspection revealed one or several microsomes as a consequence of rapeseed oil feeding. After about 200 hours more and more yeast cells changed aspect from the normal round shape to a flattened shape. This forms an indication of the stress the cells were experiencing due to the product formed and or the decreasing glucose concentration.

The final sophorolipid yield amounted 239.2 g (79.7 g/l) and only 0.5 g/l of dodecyl glutarate was left at the end of the experiment residual carbon. Somewhat less than 75% of the added ester was incorporated into sophorolipids, resulting in a HPLC chromatogram very similar to the one in ex.1. Besides incorporation of the ester, also de novo synthesised sophorolipids were found with an unsaturated hydrocarbon chain having the usual chain length of between 16 and 18.

### Example E.4. Alkaline hydrolysis of the sophorolipids of Example E.3.

The sophorolipid mixture obtained in example E.3. was dissolved in water (18,7 g in 100 ml AD) and titrated with KOH_{50%} at room temperature. The mixture was left to react during three days each time raising the pH with additional KOH (totalling 9,6 g KOH_{50%}), since the pH dropped twice overnight to pH 8,2. The mixture was neutralised with glacial acid to pH 7.3 and centrifuged (5 min at 16000g) to remove turbidity, probably caused by the presence of glutaric acid released during hydrolysis.

A RP-HPLC-ELSD analysis (see figure 2) revealed a clear shift to the left, indicating de-acetylation and hydrolysis of the shielded ester. The chromatogram showed one major peak (at 16 min RT) corresponding to the deacetylated SL12. The smaller peaks correspond to the retention times of hydrolysed native, de novo synthesised sophorolipids and other species further described in Example E.8. below.

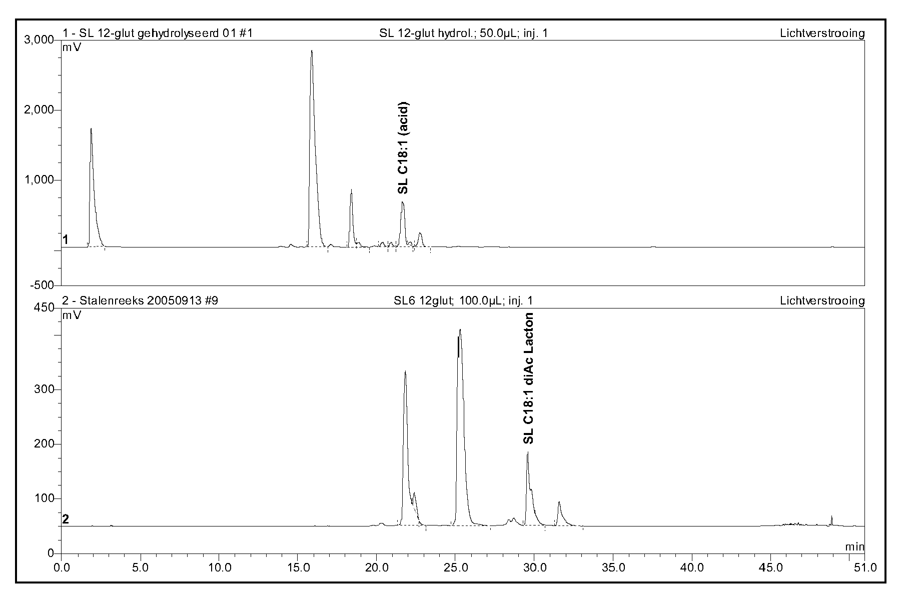

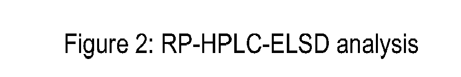

### Example E.5 Pentenyl dodecanoate fermentation in fermentor vessel.

Fermentation with pentenyl dodecanoate (ester T5 in table 2) was started similarly as disclosed in Example E.3 in respect of dodecyl glutarate. After 126, 226 and 344 hours 100 ml glucose solution was added (75 g/100 ml). It was possible to run the fermentation for a very long time without cell mortality, the pH remaining low and glucose continuously being consumed. At the end of the fermentation the residual substrate amounted 36 g/l, and 83 g/l of sophorolipids had been produced. The sophorolipids produced were identified as the lactonic and the acid diacetylated sophorolipids of the hydroxlated ester. The main new peaks however were even more hydrophilic and occurred at 15 minutes or even prior to that, indicating even shorter sophorolipids were formed as discussed in detail in Example E.8.

### Example E.6 Lauryl-myristyl malonate fermentation in fermentor vessel.

Fermentation with lauryl-myristyl malonate (ester T13 in table 2) was started as disclosed in Example E.3. After 161 hours 100 ml glucose solution was added (75 g/100 ml). Dissolved oxygen concentration decreased periodically upon each substrate addition, always remaining well above 40% From 90 hours onwards new sophorolipid types were formed with a pronounced new peak at 26,4 minutes retention time (extracted with both ethyl acetate and ethanol) and a peak complex (only extracted with ethanol). Two main peaks (at 17,7 and 19,3 minutes of retention time) are believed to be acid sophorolipids of lauryl malonate and myristyl malonate.

Sophorolipids were obtained in high yield, 55 g/l in 166 hours.

### Example E.7. HPLC-MS identification.

The sophorolipids produced in example E.3. and example E.5. (resp. from dodecyl glutarate and pentenyl dodecanoate) were extracted with both ethyl acetate and ethanol. Part of the obtained material was hydrolysed using NaOH as described in ex. E.4. and both the product resulting from the fermentation and after alkaline hydrolysis were analysed by RP-HPLC-MS (Waters ZQ) using quadrupole electrospray negative ion detection. This resulted in the following table for the hydrolysates :

**Table 4.**

| Retention time (min.) | Formula and molecular weight of identified peaks | | Identified structure in alkaline hydrolysate |
|---|---|---|---|
| | Pentenyl dodecanoate used as substrate (ester T5) | Dodecyl glutarate used as substrate (ester T0) | |
| 4,33 SF 6.8 | C5 0-Ac/OH (428) | | 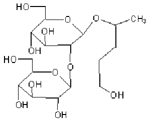 |
| 8,11 SF 10.6 | C5 0-Ac/CH3 (412) | | 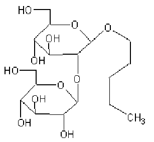 |
| 8,57 SF 11.1 | C5 0-Ac/CH3/1 glucose (250) | | 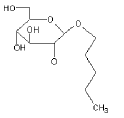 |
| 11,27 SF 13.8 | | C12 0-Ac/OH/4glucose (850) | 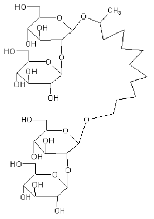 |
| 12 SF 14.5 | | C12 0-Ac/OH/3glucose (688) | 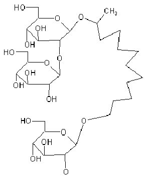 |
| 13,08 SF 15.6 | C12 0-Ac/OOH (540) | | 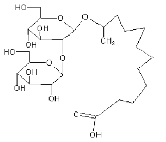 |
| 13,35 SF 15.9 | | C12 0-Ac/OH (526) | 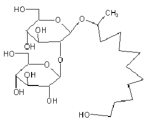 |
| 14,47 SF 17.0 | | C12 0-Ac/OH/1glucose (364) | 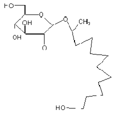 |
| 17,05 SF 19.6 | C16:1/0 Ac/OOH (594) | C16:1/0 Ac/OOH (594) | 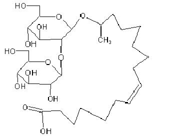 |
| 17,63 SF 20.1 | C16:0/0 Ac/OOH (596) | C16:0/0 Ac/OOH (596) | 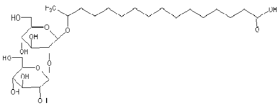 |
| 19,06 SF 21.6 | C18:1/0 Ac/OOH (622) | C18:1/0 Ac/OOH (622) | 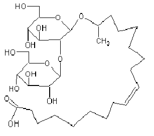 |
| 19,87 SF 22.4 | C18:0/0 Ac/OOH (624) | C18:0/0 Ac/OOH (624) | 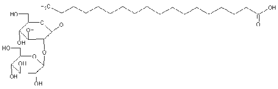 |

In both examples de novo synthesis of standard sophorolipids was observed. These sophorolipids were detected at retention times between 17-20 minutes and have molecular weights ranging from 594 to 624 Dalton. Pentenyl dodecanoate yielded a substantial amount of de novo sophorolipids, whereas dodecyl glutarate yielded only a small amount of them (see Example E.1).

Both esters yielded the more hydrophilic sophorolipids species that were expected. As evidenced by this table the use of directional shielded esters (as in dodecyl glutarate) forces Candida to hydroxylate opposite to the hydrophilic terminus and results in a mixture of SL12 species as opposed to the use of the non directional shielded ester pentenyl dodecanoate which results in both SL5 and SL12 species, mainly SL5. The pentenyl dodecanoate resulted among others in pentane-sophorolipids (412 dalton at 8.11 min), the relative peak area suggesting this unexpected sophorolipid to be preferentially synthesised, especially since it's less ionisable than SL-pentanol (with an extra hydroxyl) and SL-dodecanoic (with a free carboxyl group). Possibly dodecanoic acid acts as a carrier for the pentanol, which is subsequently bound to the first glucose unit without being hydroxylated.

Both esters furthermore yielded trace amounts of sophorolipids with 1, 3 and 4 glucose units, as described by Brakemeier and co-workers (1995). The hydrophilic nature of these sophorolipids at both ends of the molecular weight range obviously depends on the number of glucose units.

### Example E.8. Shakeflask fermentations on the other esters.

Shakeflask experiments with dodecyl pentanoate (ester T2 in table 2) showed a pronounced substrate toxicity from 10 mM onwards, probably caused by residual solvent. A new T2 batch was produced with increased care to completely remove the solvent. This batch was not toxic, with the number of CFU remaining stable (above 10⁹/ml) over time, independent of the substrate concentration.

The mass spectrometric analysis of the peaks identified after alkaline hydrolysis of sophorolipids obtained from T2 ester, other than the obvious de novo sophorolipids and sophorolipids obtained from hydroxy dodecanoic acid is shown in table 5 below. The T2 ester yields less C₅ species than when using the T5 ester, but still the absence of a directional hydrophilic terminus such as in the T0 and T13 ester allows for hydrolyxation at both ends yielding both C₅ and C₁₂ species. Note that again methyl ended sophorolipids as well as sophorolipids with surplus glucose units were identified.

Shakeflask experiments with decyl heptanoate (ester T10 in table 2) gave similar results, indicating high toxicity for the first ester batch. This time however the improved batch remained slightly toxic, the number of CFU being reduced from 6,5 x 10⁹ to 3 x 10⁹ CFU/ml. The mass spectrometric analysis after alkaline hydrolysis of sophorolipids obtained from T10 ester, other than the obvious de novo sophorolipids and hydroxy dodecanoic acid is summarized in table 6 below. Basically these results confirm the T0 experiment and illustrate that it is possible to incorporate other chain lengths than C16/18 in a sophorolipid, and that it is possible to work with a technical C12-14 fatty moiety mixture. Note that again methyl terminated sophorolipids as well as sophorolipids with surplus glucose units were identified as de novo synthesized products. For both esters the dissolved oxygen concentration never came below 40% and averaged 65% (T2) and 63% (T10).

**Table 5: Mass spectrometric analysis of sophorolipids obtained from T2 ester.**

| Retention time (min.) | Formula and molecular weight of identified peaks | | Identified structure of alkaline hydrolysate |
|---|---|---|---|
| | Dodecanol pentanoate used as substrate | Pentenyl dodecanoate used as substrate (T5 ex.E.8) | |
| 4.33 | | SL₅ 0-Ac/OH (428) | 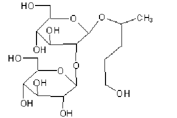 |
| 4.95 | SL₅ 0-Ac/OOH (442) | | 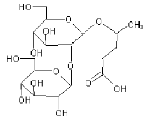 |
| 8,11 | | SL₅ 0-Ac/CH₃ (412) | 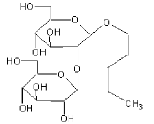 |
| 8,57 | | SL₅ 0-Ac/CH₃/1 glucose (250) | 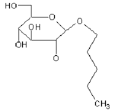 |
| 13,08 | | SL₁₂ 0-Ac/OOH (540) | 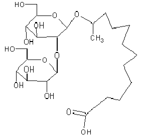 |
| 13.73 | SL₁₂:0,-Ac/OH (526) | | 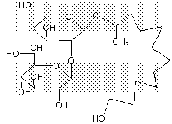 |
| 19.70 | SL₁₂ 0 Ac/CH₃ (510) | | 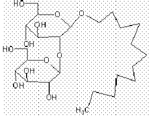 |

**Table 6: Mass spectrometric analysis of sophorolipids obtained from T13 ester.**

| Retention time (min.) | Formula and molecular weight of identified peaks | | Identified structure of alkaline hydrolysate |
|---|---|---|---|
| | Lauryl malonate substrate (T13) | Dodecyl glutarate as substrate (T0 ex.E.7) | |
| 11.35 | SL₁₂ 0-Ac/OH/4glucose (850) | SL₁₂ 0-Ac/OH/4glucose (850) | 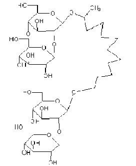 |
| 12.08 | SL₁₂ 0-Ac/OH/3glucose (688) | SLi₂ 0-Ac/OH/3glucose (688) | 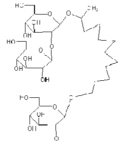 |
| 13.19 | SL₁₄ 0-Ac/OH/4glucose (878) | | 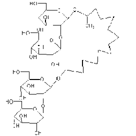 |
| 13.38 | SL₁₂ 0-Ac/OH (526) | SL₁₂ 0-Ac/OH (526) | 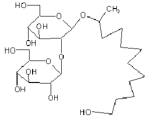 |
| 14.03 | SL₁₄ 0-Ac/OH/3glucose (716) | | 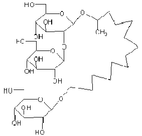 |
| 14.47 | | SL₁₂ 0-Ac/OH/1glucose (364) | 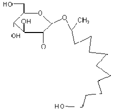 |
| 15.68 | SL₁₄ 0-Ac/OH (554) | | 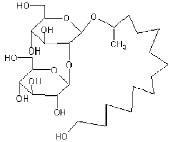 |
| 19.83 | SL₁₂/0 Ac/CH₃ (510) | | 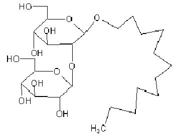 |

### Ex. 0.1. Ozonolysis of commercially available sophorolipids.

Sopholiance was obtained from Soliance (Reims, France). The product contains approx. 50% sophorolipids, the GC-MS fatty acid spectrum of which is shown in the table below, as well as approx.10% residual carbon source. 800 gram of product was hydrolysed by adding KOH_{100%} until the pH remained stable above pH 9, which required approx.70 g KOH.

| | % |
|---|---|
| C18dien | 2,02 |
| C18en | 9,68 |
| 15HOC15 | 1,06 |
| 16HO16en | 0,24 |
| 16HOC16 | 1,73 |
| C20en | 0,31 |
| C20trien | 0,24 |
| 17HOC18dien | 3,86 |
| 17HOC18en | 49,66 |
| 17HOC18 | 3,13 |
| 18HOC18dien | 10,33 |
| 18HOC18en | 12,9 |
| 18HOC18 | 0,3 |
| C22en | 0,17 |
| Unidentified substance | 0,24 |
| Phthalate | 0,29 |
| 19HOC20en | 0,22 |
| Unidentified substance | 0,2 |
| 24-Methyl-cholesta-5,22- | |
| dien | 0,38 |
| 24-Methyl-cholest-5-en | 1,37 |
| 24-Ethyl-cholest-5-en | 1,67 |

H₂O₂ (35%) addition to the hydrolysed mixture did not result in new compounds as monitored by RP-HPLC-ELSD, the methodology of which is described by Fleurackers (2003). New peaks appeared however when the temperature was raised. This was probably due to epoxidation of the double bond by organic peracids formed in the presence of H₂O₂ (CH₃-COOH + H₂O₂ → CH₃-COOOH + H₂O) (see Organic Chemistry, S. Ege, 3rd ed., pp. 335-337). Because the conditions of the ozonolysis did not involve an equally raised temperature, this epoxide formation was not further investigated.

So as an alternative way of oxidative cleaving, ozonolysis was performed at room temperature by sparging ozone enriched (0.4% O₃, ^{v}/ᵥ) oxygen (Messer, grade 4.5) through sintered glass in an aqueous solution (800 ml) of the hydrolysed sophorolipids, the flow rate depending on foam formation (3-100 L gas mixture per hour). The ozone was generated with an Ozomatic ozone generator (Anseros), ozone concentration was monitored using a Merck test kit and residual ozone was catalytically destroyed.

When operating at pH 4 no reaction was observed after 2 days, probably due to low sophorolipid accessibility by the ozone. At pH 5 the solution becomes clear and starts to foam. At even higher pH the foam becomes stable and may hinder the reaction. The optimal pH for this reaction proved to be 5.5 thus keeping the balance between sufficient solubility and foamability

In order to compensate for the acidification (with approx. 0.4 pH units) during ozonolysis, the optimal pH was set at pH 5.5 using acetic acid. After 7 hours the reaction, during which temperature rose with 20°C, was complete as evidenced by RP-HPLC-ELSD and temperature monitoring, the temperature returning back to 20°C. Upon completion of the reaction the pH was raised to 9 by addition of KOH in order to destroy any peroxides that may have been formed. The ozonolysis reaction mixture was analysed by RP-HPLC-MS (Waters ZQ) using quadrupole electrospray negative ion detection. Table 7 and chromatogram below list the major components found, the main component being saturated SL9:0 with a terminal carboxyl group, as well as dicarboxylic acids and traces of saturated SL16 and SL18, which are insensitive to ozonolysis. Table 7 ranks the components according to their retention time to allow easy interpretation of the chromatogram.

| M.M. (Da) | Peak label on chromatogram | Retention time (min) | Chemical nature and origin | Molecular structure |
|---|---|---|---|---|
| 498 | SL C₉:0 | 7,6-12,6 | Pelargonic SL, ozonolysate of SL C₁₈:1 | 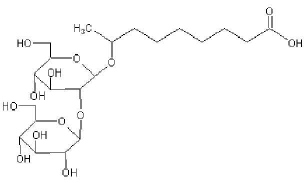 |
| 188 | Azelaic | 13,7 | Diacid (C₉), ozonolysate of SL C₁₈:1, C₁₈:1 and C₁₈:2 | 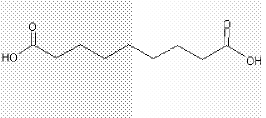 |
| 216 | Undecane diacid | 17,6 | Diacid (C₁₁), ozonolysate of residual C_{20:1} rapeseed FA | 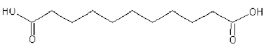 |
| 596 | SL C₁₆:0 | 19,6-20,6 | Palmitic SL, alkaline hydrolysed saturated SL | 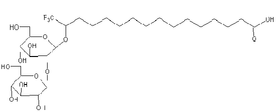 |
| 610 | n.a. | 21,1 | C₁₇:0 SL, alkaline hydrolysed saturated SL | 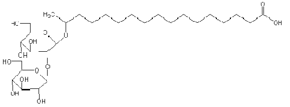 |
| 624 | SL C₁₈:0* | 22,6-23,1 | Stearic SL, alkaline hydrolysed saturated SL | 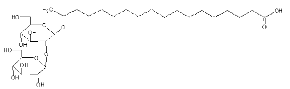 |
| 158 | Pelargonic | 23,9 | Fatty acid (C₉:0), ozonolysate of C_{18:1} fatty acid | 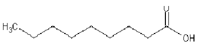 |
| 298 | n.a. | 30,6-32,6 | Hydroxy oleic acid, acid hydrolysed SL_{18:1} | 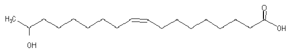 |
| 300 | n.a. | 30,6-32,6 | Hydroxy stearic acid, acid hydrolysed SL_{18:0} | 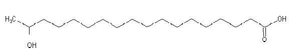 |
| 256 | n.a. | 37,6 | Fatty acid (palmitic), residual saturated FA | 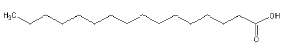 |
| 282 | n.a. | 38,0 | Fatty acid (oleic), residual unsaturated FA | 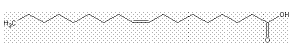 |
| 284 | C₁₈:0 | 40,6 | Fatty acid (stearic), residual saturated FA | 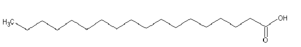 |

The chromatogram below illustrates Sopholiance in the upper chromatogram, hydrolysed sophorolipids in the central chromatogram to the ozonolysed mixture in the lowest chromatogram, prior to any purification.

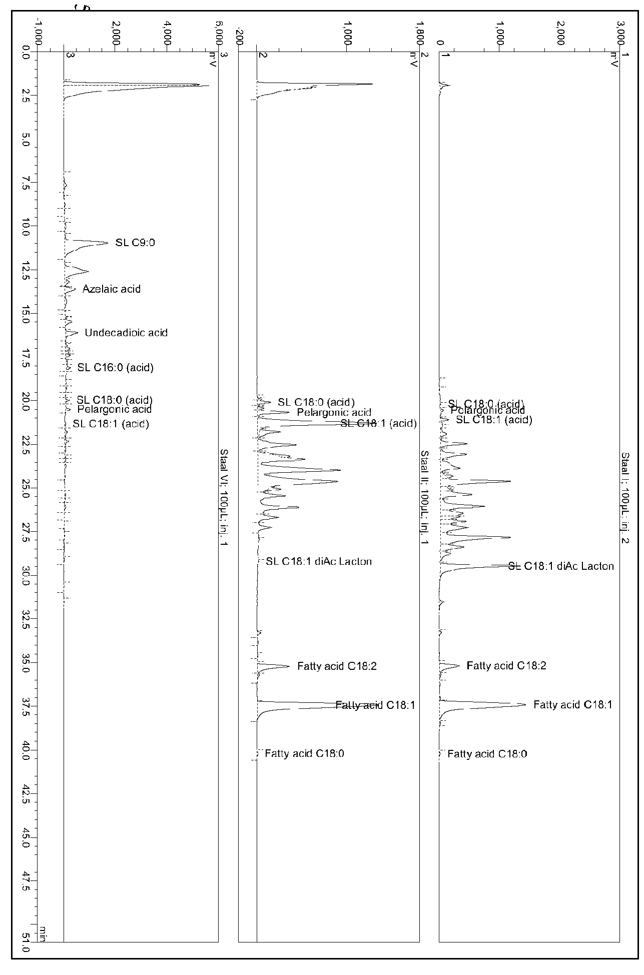

Unlike the conclusions of Wang and coworkers (1998) the inventors have observed that β-D-aldosidic linkage between the two glucose units was not attacked by ozone, since no mono-glucose components were identified.

H₂O₂ (35%) addition to the ozonolysed mixture in order to perform an oxidative work-up of any residual ozonides proved unnecessary since H₂O₂ is automatically formed when ozone is sparged through a aqueous solution.

At 150 mbar and 65°C water was removed from the reaction mixture to the extent possible. An oily layer on top of the condensate was observed, which proved to be mainly pelargonic acid. Azelaic acid however could not be removed because of its high boiling point. The residue was dissolved in 95% ethanol at 60°C and allowed to cool to room temperature, during which a white crystalline precipitate was formed. This was filtered off, washed with ethanol and dried to obtain 139 g dry matter. HPLC analysis revealed that the described purification method removes most of the acetate salts formed (as shown in the table below), but was less successful in removing pelargonic and azelaic from the ozonolysed sophorolipids (SL 9:0).

| | Salt (front peak) | | SL C₉:0 | | Azelac | | Undecane diacid | |
|---|---|---|---|---|---|---|---|---|
| | Meas. | **Norm.** | Meas. | **Norm.** | Meas. | **Norm.** | Meas. | **Norm.** |
| Before | 1500 | **231** | 649 | **100** | 365 | **56** | 94 | **14** |
| After | 245 | **125** | 196 | **100** | 68 | **35** | 17 | **9** |

| | | | | | | | | |
|---|---|---|---|---|---|---|---|---|
| Meas.: measured peak area Norm.: normalised with respect of the measured value for SL C₉:0 | | | | | | | | |

### Ex.O.2 Alkaline ozonolysis on a crude sophorolipid mixture obtained from rapeseed oil.

A crude sophorolipid mixture was obtained from the cultivation of Candida bombicola ATCC 22214 in fed batch in a Biostat D100 at 30°C on rapeseed oil and glucose. Alkaline hydrolysed sophorolipids (cf Ex.O.1) were treated with ozone at pH 12,5 to study the effect of the absence of H₂O₂ since peroxides degrade in alkaline media. Furthermore the effect on glycosidic bonds was monitored.

As expected abundant foam was formed, which necessitated the gas flow to be reduced to 10^{L}/ₕ. After about 6 hours the solution was clearly bleached and a temperature rise was observed although less pronounced (up to 30-35°C) than with ozonolysis at pH 5.5. After 6 hours the reaction was stopped. An HPLC analysis (not shown) revealed that the sophorolipids had hardly reacted with ozone. Three new peaks appear at RT 34, 36 and 38.5 minutes, corresponding to free fatty acids resp. C_{18:3}, C_{18:2} en C_{18:1} formed by slow alkaline hydrolysis of residual oil present in the sophorolipid mixture. A GC-MS analysis did not show any HFA being formed.

### Example 0.3. Ozonolysis on a crude sophorolipid mixture obtained from rapeseed oil.

Example O.1. was repeated starting from 313 gram crude sophorolipid mixture obtained from the cultivation of Candida bombicola ATCC 22214 in fed batch in a Biostat D100 at 30°C on rapeseed oil and glucose. In order to determine the exact amount of sophorolipid in the starting material, a Dean-Stark destillation with toluene was performed until the condensate did not contain anymore water. After toluene evaporation 223 g dry weight was recovered (approx. 71 % dry mass). This material was re-dissolved in 0.5 litre of water and titrated with KOH. Adding 57.2g of KOH and keeping the solution for 2 hours at 70°C resulted in a stable pH of 12,7. Hydrolysis was considered complete since no further pH drop was observed. The added amount of KOH closely matches the theoretical demand (54.5 g). The mixture was diluted to 1.25 l water and the pH was adjusted to 5.5 by adding 23.5g acetic acid. This solution was sparged for six hours with an O₂/O₃ gas mixture (flow rate: 100L/h, ozone production: 44mgO₃/L). Finally pressurised air was used to purge any residual ozone. The pH had dropped to 5.07. The reaction mixture was turbid and after standing overnight a clear sedimentation had occurred.

The precipitate was removed using centrifugation and the supernatant was glass-filtered. Since further sedimentation was observed in the filtrate, the procedure was repeated after which the filtrate remained clear. The solid fractions were collected, pooled and homogenised in 0.5 l water, which was subsequently adjusted to pH 12.5 with KOH_{50%}, which proved efficient in solubilising the complete solid fraction *('Precipitaat @ pH5').*

The pH was lowered to 2 by adding HCl_{37%}- Overnight a precipitate formed which could completely be removed by filtration. Again the residue was redissolved in 250 ml of water at pH 12.5). These precipitates were shown to be azelaic acid, the first precipitate forming in the pH range between pKa₁ and pKa₂ of azelaic acid, where one carboxyl group is still ionised. At the pH where the second precipitate is formed, both carboxyl groups showed to be protonated. This may explain why at pH 5 part of the azelaic acid remains in solution, only to sediment at pH 2.

The reaction mixture after double acid precipitation was adjusted with KOH to a neutral pH in order to spare the glucosidic bonds when removing water. Methanol was added to remove a white crystalline precipitate (potassium acetate and chloride), which was removed by filtration to leave an amber viscous liquid (SL9:0). Methanol was removed through distillation. The residue was re-dissolved to yield a 50 % product in water. The HPLC chromatogram below elucidates this process, starting from crude lactonic sophorolipids, over hydrolysis and ozonolysis to the precipitate on one hand and the purified SL9:0 fraction on the other hand. It would seem that most of the hydrolysed ozone-insensitive (i.e. saturated) sophorolipids were removed together with the azelaic acid.

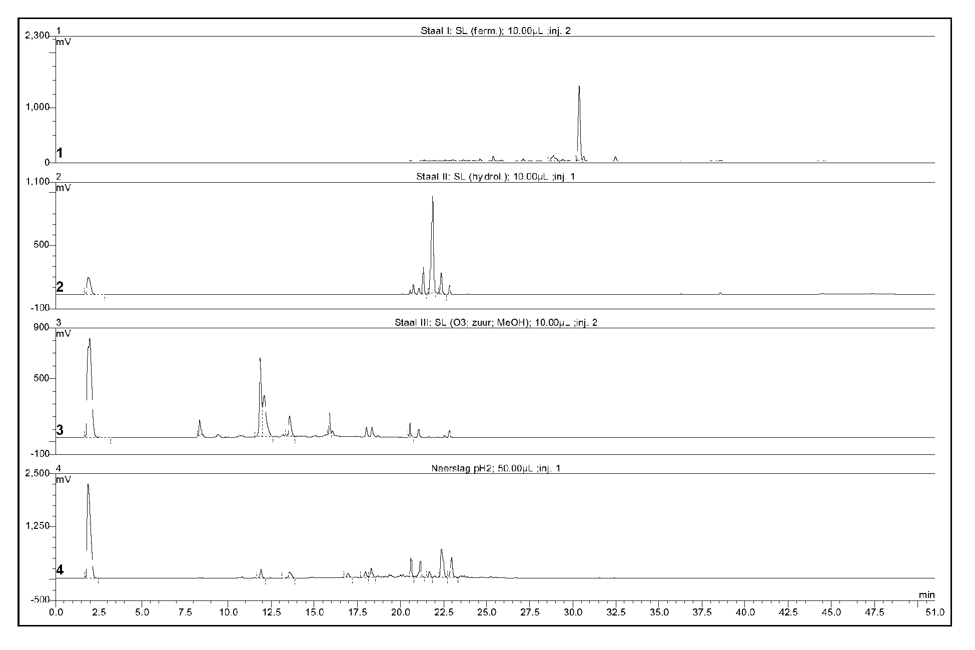

### Ex.0.4 Ozonolysis on a crude sophorolipid mixture obtained from sunflower oil.

Experiment Ex.O.1 was repeated using 110 gram crude sophorolipids obtained from technical grade sunflower oil added from the start to a Biostat B5 Candida bombicola culture. Similar to the previous examples these crude sophorolipids were dissolved in 375 ml of water, titrated with KOH and subjected to ozone as described above.

HPLC analysis of the ozonolysate revealed an additional more hydrophilic peak (likely to be SL7:0, with a RT of about 8 min.). This was to be theoretically expected since sunflower oil contains a substantial amount of multiple unsaturated C18 (esp. C18:2).

### Example 0.5. Acid hydrolysis of C₁₈ sophorolipids from rapeseed oil.

The alkaline hydrolysed C18:1 sophorolipids obtained from Ex.O.3. were boiled for 4 hours at a pH below 0.5 (HCl). The solution became turbid and split into two layers upon cooling. The upper layer was extracted with diethylether and submitted to an HPLC analysis which revealed three peaks at RT 34, 36 and 38.5 minutes corresponding to free fatty acids resp. C_{18:3}, C_{18:2} en C_{18:1} as in Ex.O.2. A new peak corresponding to hydroxyl oleic acid appeared at 31 minutes.

Alternatively acid hydrolysis with acetic acid at pH 4.5 did not cause any significant change in the sophorolipid spectrum and no hydroxyl oleic acid was formed.

### Example 0.6. Acid hydrolysis of SL9:0.

Purified SL9:0 material obtained in Ex.O.3 was subjected to hydrochloric hydrolysis as in the previous example. After 40 minutes the solution became turbid, to become clear again when the pH was raised to 9.5, corresponding to the pH at which the azelaic precipitates in Ex.O.3 became clear. The acid hydrolysed material was subjected to an RP-HPLC-ELSD which indicated that all peaks of the purified SL9:0 have disappeared, apart from azelaic (at RT 13.7 min) and 3 peaks in the region 21-22.5 minutes, corresponding to the retention time region of alkaline hydrolysed saturated sophorolipids (traces of which were present in the starting material). Given the extreme pH however these peaks are very unlikely to correspond with any glycosidated material. Given the increase in their peak area it is assumed that these peaks correspond to short-chained hydroxyl fatty acids (w- and w 1 hydroxylated variants of OH-C₇, OH-C₉). Mass spectrometry is needed to confirm this.

### Example 0.7. Ozonolysis of Partially hydrolysed sophorolipids.

25g of diacetylated lactonic C₁₈:1 sophorolipids were finely dispersed in 250mL water. Using 3M KOH, 0,7 molar equivalents of KOH were added to obtain a mixture containing sophorolipids which have none, one, two or all of their esteric bonds hydrolysed. The obtained mixture is clear and has a final pH of 7,2. The reaction mixture may be heated to facilitate this reaction. Electrospray quadrupole RP-HPLC-MS analysis shows the presence of molecular species which have undergone the hydrolysis of none, one, two or three of their esteric bonds. The solution was then sparged with an O₂/O₃ mixture for 3h. Gas flow rate: 30L/h; ozone concentration: 44g O₃/m³. A notable increase in temperature was observed and when the temperature dropped back to room temperature, the reaction was taken to be complete. Electrospray quadrupole RP-HPLC-MS analysis confirmed that all the unsaturated sophorolipids were converted into their ozonolysed counterparts, showing both reductive and oxidative work-up, yielding an aldehyde or a carboxylic group, respectively. The following table shows the reaction products obtained from partial hydrolysis and subsequent ozonolysis.

As the starting material, the di-acetylated lactonic sophorolipid containing a 17-hydroxy oleic acid lipophilic moiety is used in this example. Partial hydrolysis of this sophorolipid yields six different masses, this being the combination of opening the lactone or not (two possibilities) and removing zero, one or two acetyl residues from the sophorose moiety (three possibilities). Subsequent ozonolysis of this partial hydrolysate at the stated pH will convert the double bond into either two carboxyls, a carboxyl and an aldehyde or two aldehydes. If during the previous step the lactone was opened, then part of the lipophilic moiety is removed from the sophorolipid entirely and only two resulting masses occur: terminal carboxyl or aldehyde. If the lactone was not opened, then three resulting masses occur: two carboxyls, a carboxyl and an aldehyde or two aldehydes.

| Starting material | | After partial hydrolysis | | After ozonolysis | |
|---|---|---|---|---|---|
| Mol. Mass | Structure | Mass in MS analysis | Assigned structure | Mass in MS analysis | Assigned structure |
| 688 | 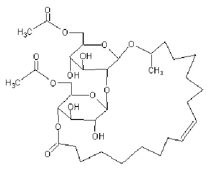 | 622 | 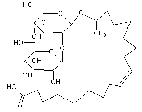 | 482 | 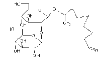 |
| | | | | 498 | 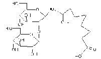 |
| | | 664* | 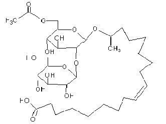 | 524* | 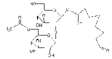 |
| | | | | 540* | 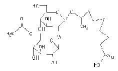 |
| | | 706 | 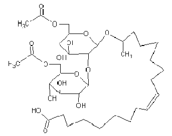 | 566 | 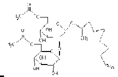 |
| | | | | 582 | 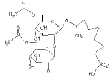 |
| | | 604 | 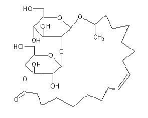 | 636 | 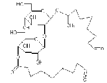 |
| | | | | 652 | 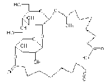 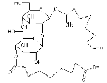 |
| | | 646* | 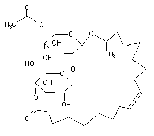 | 678* | 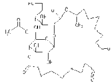 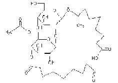 |
| | | | | 694* | 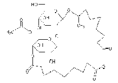 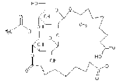 |
| | | | | 710* | 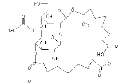 |
| | | 688 | 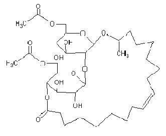 | 720 | 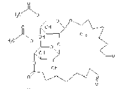 |
| | | | | 736 | 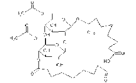 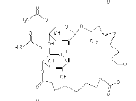 |
| | | | | 752 | 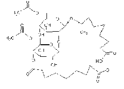 |

(*): Only one of two possible isomers due to the possibility of either acetylation at carbon 6' or carbon 6" is depicted.
Thus from this experiment it can be concluded that the pH at which the ozonolysis is performed determines the nature of the terminal group of the ozonolysate. A pH of approximately 7 yields a partially reductive workup to an aldehydic end group. Moreover ozonolysis of partial hydrolysates allows obtaining partially acetylated short chain sophorolipids.

### Example A.1. Surface activity of a mixture of azelaic acid and SL9:0.

The product obtained from the ozonolysis as disclosed of example O.1., which contained a substantial amount of azelaic acid was tested for its surface activity using the pendant drop method on a DSA100S (Krüss gmbH). The surface tension was reduced to 30mN/m, which is indicative for an efficient surfactant.

The dynamic surface tension was determined with a bubble tensiometer (Sita GmbH) at 29ms and compared to commercially available Sopholiance (Soliance). A faster decrease in the surface tension was observed with the sophorolipid of the present invention that had been subjected to ozonolysis, as compared to Sopholiance. This suggests that the sophorolipid of the present invention which has been subjected to ozonolysis is an efficient wetting agent or a surfactant that is well suited for dynamic conditions, industrial floor cleaning, window cleaning, dishwashing, pesticide spraying emulsions etc. SL9:0 presents the additional advantage of showing low foaming.

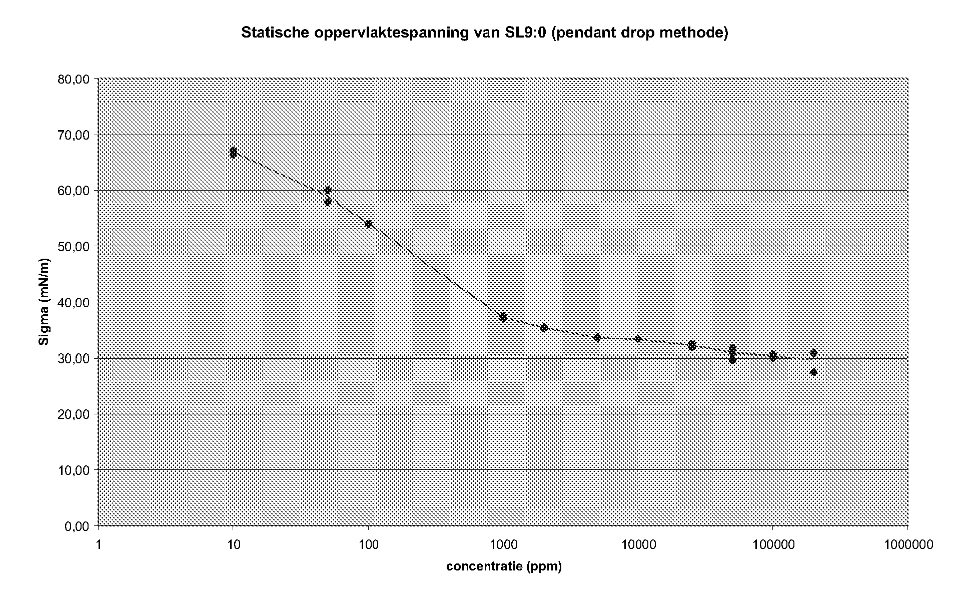

### Example A.2. Performance of the mixture of azelaic acid and SL9:0 as a wetting agent.

The contact angle of the sophorolipid obtained after ozonolysis as disclosed in example O.1. was measured on PVC at 20°C with a DSA 100S instrument (Krüss Gmbh) and compared to those of Simulsol AS48 (ethylhexylglucoside from Seppic). At a concentration of 100 ppm of the surfactant there is hardly any difference between the two, at 250 ppm the contact angle of the surfactant of this invention is 5° lower, implying a better wetting, and thus a better cleaning, drying etc. At higher concentrations the difference mounts to 17°.

**Table 8.**

| Simulsol AS48 | Contact angle on PVC | | SL C9:0 | Contact angle on PVC | |
|---|---|---|---|---|---|
| Concentration (ppm) | 0 | Standard deviation | Concentration (ppm) | | Standard deviation |
| 0 | 107.3 | 1.86 | 0 | 107.3 | 1.86 |
| 10 | 101.6 | 1.2 | 10 | 106.7 | 1.56 |
| 50 | 103.1 | 0.92 | 50 | 105.3 | 0.57 |
| 100 | 102.8 | 0.75 | 100 | 103.15 | 1.06 |
| 250 | 102.08 | 0.43 | 250 | 95.7 | 0.29 |
| 1000 | 95.3 | 3.54 | 1000 | 72.05 | 1.33 |
| 2000 | 89.4 | 0.31 | 2000 | 65.46 | 2.54 |
| 5000 | 64.4 | 1.48 | 5000 | 54.77 | 0.99 |
| 10000 | 41.8 | 1.97 | 10000 | 50.9 | 1.31 |

### Example A.3. Hydrotrope efficiency of the mixture of azelaic acid and SL C9:0.

The product of example O.1. which still contains an amount of azelaic acid was tested for its performance as a hydrotrope in conditions corresponding to alkaline bottle cleaners and similar industrial cleaning products. In this example the amount of hydrotrope needed to clarify a 100 ml AD solution of 2.5 wt. % laureth sulphate and 10% NaOH. The results are given in the table 9 below.

**Table 9.**

| hydrotrope | Chemical nature | % active matter needed |
|---|---|---|
| Simulsol AS48 (Seppic) | Ethyl hexyl glucoside | 0.38 |
| Simulsol SL4 (Seppic) | Butyl glucoside | 12 |
| Azelaic SL C9:0 | Mixture of azelaic acid and SL C9:0 | 0.68 |
| Ethanol | Ethanol | 26 |
| Manro SXS (Manro) | Sodium xylene sulphonate | 27 |
| Sopholiance (Soliance) | Native sophorolipid mixture | 1.08 |

These data suggest the impure SLC9:0 to be a much more efficient hydrotrope than xylenesulfonate and comparable to Simulsol AS48 and Sopholiance, although the latter is instable in alkaline conditions.

### Example A.4 Hydrotrope efficiency of the mixture of azelaic acid and SL C9:0.

The product of example O.1. which still contains an amount of azelaic acid was tested for its performance as a hydrotrope in conditions corresponding to domestic laundry products that are highly concentrated in nonionic surfactants. The viscosity of a 100 ml AD solution of 35% Laureth 7 10 % sodium alkylbensene sulphonate and 10 % of hydrotrope (always as % active matter) was determined as a measure for its efficiency as a hydrotrope. The product of this invention appeared to be a more efficient hydrotrope then the market reference xylene sulphonate (Manro).

| hydrotrope | Chemical nature | Viscosity (mPa.s at 25°C) |
|---|---|---|
| No hydrotrope added | | 13750 |
| Simulsol AS48 (Seppic) | Ethyl hexyl glucoside | 4338 |
| Simulsol SL4 (Seppic) | Butyl glucoside | 949 |
| Azelaic SL C9:0 | Mixture of azelaic acid and SL C9:0 | 507 |
| Manro SXS (Manro) | Sodium xylene sulphonate | 1022 |
| Sopholiance (Soliance) | Native sophorolipid mixture | Extremely viscous gel |

### Example A.5. Stability of the sophorolipid of the present invention in cleaning products.

The physical stability of sophorolipids in solution was determined at 40°C both at pH 6 and 8 in combination with 6% Glucopon 600 (Cognis) either with or without additional surfactants. Texapon 842 (40% octylsulfate, Cognis), Sopholiance (65% sophorolipid mixture, Soliance,), Simulsol SL4 (50% butylglucoside, Seppic), Simulsol AS48 (65% ehtylhexylglucoside, Seppic), AG6206 (75% hexylglucoside, Akzo Nobel), AG6210 (60% C8-10 glucoside Akzo-Nobel), Glucopon 215 (65% C8-10 glucoside, Cognis) and SL c9:0 (50% produced according to Ex. O.2.) were tested.

**Table 10.**

| Sopholiance % | % added surfactant | pH | Stable |
|---|---|---|---|
| 2 | 0 | 6 | No |
| 2 | 1.5% Texapon 842 | 6 | Yes |
| 2 | 1 % SL9:0 | 6 | Yes |
| 2 | 2% SL9:0 | 6 | Yes |

The pH of Sopholiance based formulations at pH in excess of 7 automatically drops down to 6-6.5 because of deacetylation of the sophorolipids. At pH 6 a second surfactant is needed to keep the product stable. Partial ozonolysis may improve sophorolipid stability, as suggested by the latter two formulations.

**Table 11.**

| SL9:0 % | % added surfactant | pH | Stable |
|---|---|---|---|
| 2.4 | 0 | 6 | No |
| 1 | 1.5% Texapon 842 | 6 | Yes |
| 2.4 | 1.5% Texapon 842 | 6 | Yes |
| 4.4 | 1.5% Texapon 842 | 6 | Yes |
| 2.4 | 1.5% Texapon 842 | 8 | Yes |
| 2.4 | 1 % Simulsol SL4 | 8 | No |
| 2.4 | 2% Simulsol SL4 | 8 | No |
| 2.4 | 3% Simulsol SL4 | 8 | No |
| 2.4 | 4% Simulsol SL4 | 8 | No |
| 2.4 | 0.5% Simulsol AS48 | 8 | No |
| 2.4 | 1 % Simulsol AS48 | 8 | No |
| 2.4 | 1.5% Simulsol AS48 | 8 | No |
| 2.4 | 2% Simulsol AS48 | 8 | No |
| 2.4 | 3% Simulsol AS48 | 8 | Yes |
| 2.4 | 2% AG6206 | 8 | No |
| 2.4 | 3% AG6206 | 8 | No |
| 2.4 | 4% AG6206 | 8 | No |
| 2.4 | 2% AG6210 | 8 | No |
| 2.4 | 3% AG6210 | 8 | Yes |
| 2.4 | 4% AG6210 | 8 | Yes |
| 2.4 | 2% Glucopon 215 | 8 | Yes |
| 2.4 | 3% Glucopon 215 | 8 | Yes |
| 2.4 | 4% Glucopon 215 | 8 | Yes |

As evidenced by table 11, SL9:0 needs to be combined with a second surfactant to obtain a stable formulation. Octylsulphate or C8-10 alkylpolyglucosides are well suited for stabilisation at pH 6 and 8.

### Example A.6. Hard surface cleaning efficiency of SL C9:0 at pH 6.

The product produced in Example O.2. was purified and was tested in combination with various surfactants for its hard surface cleaning efficiency at pH 6 in concentrated applications by measuring the colour value Y, i.e. the reflectance of alight beam directed on the substrate to be cleaned. Experimental cleaning was done according to the IPP test (Institut for Putz und Pflegemittel) test (Kiewert, 1981). PVC strips (4 per formulation) are soiled with a 150 micrometer layer of a mixture of carbon black and mineral oil. The mixture was left to air dry for 60 to 90 minutes, after which the strips are fixed onto a Gardner apparatus (Washability Model 1721, Braive Instruments). Then, 10 ml of the detergent solution was applied on a sponge and an additional 10 ml is directly applied on the PVC strips. Subsequently the sponge was made to move back and forth over the PVC strips using a Superchroma reflectometer and averaged for both PVC strips.

The formulations tested are typical all purpose cleaners, apart from the pH 6 which is kept lower than the typical value of 8-8.25 because of stability reasons as explained in example A.5. Surfactant mixtures were used (amounts given in parts by weight) to improve product stability. Glucopon 600 (50%) and Texapon 842 (40%) were purchased from Cognis GmbH, Sopholiance (65%) from Soliance. Soap (25% potassium coconut oleic) was produced at Ecover, SLc9:0 (50%) was synthesized according to example O.2. above.

| | Composition in parts by weight | Active matter % | Y-value |
|---|---|---|---|
| 1 | 8 Glucopon + 2.14 Texapon + 1.6 soap | 5.3 | 21.09 |
| 2 | 2 Sopholiance + 6 Glucopon + 1.5 Texapon + 0.8 soap | 5.1 | 23.42 |
| 3 | 2,4 SL9 + 6 Glucopon + 1.5 Texapon + 0.8 soap | 5.0 | 20.26 |
| 4 | 1 SL9 + 2 Sopholiance + 6 Glucopon + 0.8 soap | 5.0 | 30.61 |
| 5 | 2 SL9 + 2 Sopholiance + 6 Glucopon + 0.8 soap | 5.5 | 30.3 |
| 6 | 12 Glucopon + 2.5 Texapon + 1.5 soap | 7.4 | 26.26 |
| 7 | 2.4SL9 + 6 Glucopon + 0.8 soap | 4.4 | 36.87 |
| 8 | 2.4SL9 + 6 Glucopon | 4.2 | 35.22 |
| 9 | 2 Sopholiance + 6 Glucopon (unstable at 40°C) | 4.3 | 44.09 |

From the table above it appears that by using sophorolipids that have been subjected to ozonolyse it's possible to outperform high surfactant concentrations. The combination with Texapon 842 seems detrimental to hard surface cleaning performance.

### Ex.A.7 Hard surface cleaning efficiency of SL9:0 at pH6 and 8

The product of Ex.0.3, which is purified SL9:0, was tested in combination with various surfactants for it's hard surface cleaning efficiency according to the protocol used in the previous example, but only 2 duplicates per formula. The formulations tested are equally concentrated, the concentration being typical for all purpose cleaners. Combinations were made in such a way as to guarantee a stable product both at 4 and 40°C. Glucopon 600 (50%), Glucopon 215 (65%) and Texapon 842 (40%) were purchased from Cognis GmbH, Sopholiance (65%) from Soliance, SL9:0 (50%) was synthesised in Ex.O.3.

| Surfactant combination | pH | Y-value |
|---|---|---|
| 6% Glucopon 600 + 2.4% SL9 + 2.5% Texapon 842 | 6 | 16.5 |
| 6% Glucopon 600 + 2.4% SL9 + 2.5% Texapon 842 | 8 | 33.7 |
| 6% Glucopon 600 + 2.4% SL9 + 1.57% Glucopon 215 | 6 | 38.26 |
| 6% Glucopon 600 + 2.4% SL9 + 1.57% Glucopon 215 | 8 | 43.5 |
| 6% Glucopon 600 + 2 % Sopholiance | 6 | 41.46 |

These data again demonstrate the negative effect of Texapon 842. As expected the performance at pH 8 is better than at pH 6. Since SL9 is stable at pH 8 it's possible to formulate an even more efficient product than when using the native sophorolipids.

### Ex.A.8 Mallazzasia furfur inhibition.

The yeast *M*. *furfur* is one of the natural causes of dandruff formation. Since it is a lipophilic yeast, the in vitro growth must be stimulated by natural oils or other fatty substances when determining minimal inhibitory concentrations (MIC). We used the most common method, which is to overlay Sabouraud's dextrose agar containing cycloheximide (actidione) with olive oil.

A dilution series (1, ½ ¼, 1/8, 1/16..) was prepared. One ml of this dilution was added to 3 petri dishes per concentration and mixed with the warm agar. After cooling a drop of the yeast culture suspension was dropped on the petri dish and left to grow for 48 hours at 37°C. A visual inspection allows to determine the lowest concentration which completely inhibits growth of the yeast.

The MIC was determined for azelaic acid (pa VWR) neutralized with NaOH, azelaic mixture with SL9:0 (from ex.O.1) and calcium purified azelaic mixture with SL9:0. Whereas a 30% azelaic solution was needed for complete inhibition was needed, only 20% of the (SL9-azelaic) mixture of ex.O.1 was needed. Workup with calcium hydroxide allowed to obtain a product of which only 10% allows to completely inhibit M.furfurgrowth.

### Ex.A.9 Dynamic surface tension of SL12.

The material of ex.E.4 obtained after alkaline hydrolysis of the fermentate of dodecyl glutarate was MPLC fractionated on a Lobar Lichnoprep C18 column and the dynamic surface tension of the different fractions was determined as in ex.A.1. The best performing fraction is an alkaline stable product performing better than the hydrolysed C18 sophorolipids, but does not match the performance of the alkaline unstable native sophorolipids.

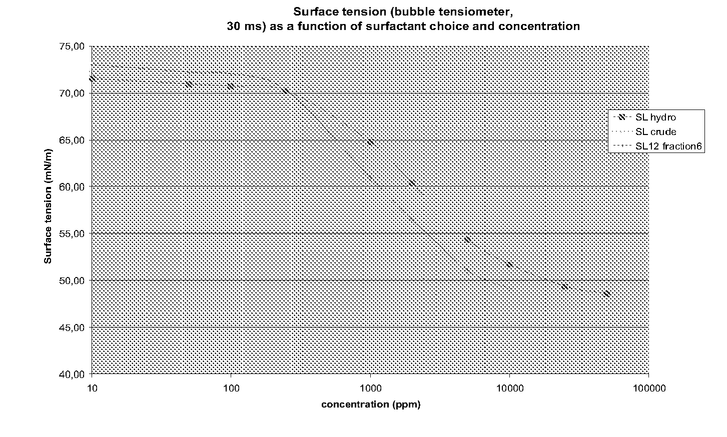

### List of references.

a) Bloodworth BC, 1995. Liquid chromatographic determination of trans-10-hydroxy-2-decenoid acid content of commercial products containing royal jelly. J AOAC Int 78: 1019-1023
b) Brakemeier A., Wullbrandt D., Lang S, 1998. Candida bombicola: production of novel alkyl glycosides based on glucose/2-dodecanol. Applied Microbiology and Biotechnology 50: 161-166
c) Cutler AJ and Light RJ, 1979. Regulation of Hydroxydocosanoic acid sophoroside production in Candida bogoriensis by the levels of glucose and yeast extract in the growth medium. J.Biol.Chem.254:1944
d) Develter D., Renkin M., Jacobs 1., 2003. Detergent Compositions. EP 1445302A1
e) Fleurackers S., 2003. HPLC analysis of sugar detergents of commercial and ecological interest. La Rivista Italiana delle Sostanze Grasse (Vol.80, juli-aug).
f) Gonzalez-Pajuelo et al, 2005. J.Ind.Microbiol.Biotechnol.32: 391-6
g) Jones D.F. and Howe R., 1968. Microbial oxidation of long-chain aliphatic compounds. Part I-V. Journal of the Chemical Society (C), p.2801-2833.
h) Lang S, Brakemeier A, Heckmann R, Spöckner S, Rau U, 2000. Production of native and modified sophorose lipids. Chemistry Today 10:76-79.
i) Lang S, Brakemeier A, Wullbrandt D, Seiffert-Störiko A, 1998. Neuartige sophoroselipide, verfahren zu deren herstellung und verwendung. PCT 9924448 nog niet gerefereerd
j) Marchal R, Vandecasteele JP, 1995. Les sophorose-lipides : propriéteés et structure. OCL. Oleagineux corps gras lipides 2(1) : 10-12.
k) Nunez A, Ashby R, Foglia TA, Solaiman DKY, 2004. LC/MS analysis and lipase modification of the sophorolipids produced by Rhodotorula bogoriensis. Biotechn. Letters 26:1087-1093.
l) Singh SK, Felse AP, Nunez A, Foglia TA, Gross RA, 2003. Regioselectiveenzyme-catalysed synthesis of sophorolipid esters, amides, and multifunctional monomers. J.Org.Chem.68:5466-5477.
m) Tulloch AP, Spencer JFT and Gorin PAJ, 1962. The fermentation of long-chain compounds by Torulopsis magnoliae. Can J Chem 40:1326-1338.
n) Tulloch AP, Spencer JFT, 1966. Fermentation of long chain compounds by Torulopsis magnoliae. III. Preparation of dicarboxylic acids from hydroxy fatty acid sophorosides. JAOCS 43:153-156
o) Tulloch AP, Spencer JFT, 1968. Fermentation of long-chain compounds by Torulopsis apicola.IV. Products from esters and hydrocarbons with 14 and 15 carbon atoms and from methyl palmitoleate. Can J Chem 46: 1523-1528
p) Wang Y, Hollingsworth Rl, Kasper DL, 1998. Ozonolysis for selectively depolymerizing polysaccharides containing β-D-aldosidic linkages. Proc Natl Acad Sci U S A. 95(12): 6584-6589
q) Wullbrandt D, Giani G, Brakemeier A, Lang S, Wagner F, 1998. Glucose- and sophorose-lipids. A Process for their preparation and their use. EP0745608
r) Zerkowski JA, Solaiman DKY, Ashby RD, Foglia TA, 2006. Head Group-Modified Sophorolipids: Synthesis of New Cationic, Zwitterionic, and Anionic Surfactants. JSD 9: 57-62

## Claims

1. A method for producing a glycolopid wherein at least one micro-organism strain capable of producing glycolipids or an acetylated derivative of a glycolipid when contacted with a carbohydrate and a hydrophobic aliphatic substrate, is contacted with at least one carbohydrate substrate and at least one hydrophobic aliphatic substrate in aqueous medium which is to be converted into at least one glycolipid, **characterized in that** as the hydrophobic hydrocarbon substrate use is made of a compound which responds to the chemical formula I or a salt or a methyl, ethyl or glycerol ester of the compound of formula I which contains at least one cleavable bond,
R¹⁰-R¹- X-Y-Z-R²-R²⁰ (formula I)
wherein
- Y is -O-, -S-, -NH-, a mono or di unsaturated bond
- X and Z may be the same or different and are chosen from the group of a - CO- group or a -CHₙ(CH₂)ₘ- group with m having a value between 0 and 4 and n between 0 and 2 which may be straight or branched
- R¹ and R² each are aliphatic hydrocarbon chains which may be the same or different chain, which may be branched or unbranched, may contain one of more unsaturated bonds and may contain one or more substituents
- R¹⁰ and R²⁰ may be the same or different and are chosen from the group of
- H, -CH₃, -CHO, -CH₂OH, -COOH, -CHS, -CH₂SH, -CN or -CH₂NR³ₚ, R³ being an aliphatic hydrocarbon chain or H, p being 1 or 2
- -R¹⁰-R¹-X is a lipophilic moiety of the glycolipid
- -Z-R²-R²⁰ is an elongator part of the compound of formula I
wherein the number of atoms in the chain determining the chain length of formula I is between 14 and 22, preferably between 16 and 18
and **in that** the at least one cleavable bond is broken to remove the elongator part from the glycolipid to obtain a short chained glycolipid.

2. The method of claim 1, **characterized in that** the hydrocarbon chain which determines the chain length of each of R¹⁰-R¹-X and -Y-Z-R²-R²⁰ contains at least 3 carbon atoms.

3. The method of claim 1 or 2, **characterized in that** R¹⁰ is -CH₃ and R²⁰ is selected from the group of a -CH₂OH, -CH₂SH, -CH₂NH₂, or - COOH group.

4. The method any one of claims 1-3, **characterized in that** the elongator part -Y-Z-R²-R ²⁰ is derived from a compound selected from the group of a dioic acid, a diol, an amino acid or a short chain hydroxyl fatty acid, and **in that** the elongator part is bound to a lipophilic moiety which is selected from the group of a fatty acid, a fatty amine, a fatty alcohol.

5. The method of claim 4, **characterized in that** the compound of formula I is a fatty alcohol esterified with a dioic acid.

6. The method of any one of claims 1-5, **characterized in that** the cleavable bond is contained in a moiety which is selected from the group of an ester, an anhydride, an amide, an ether, a thioester or a thioether and **in that** the glycolipid delivered by the micro-organism is subjected to hydrolysis in an alkaline aqueous reaction medium to hydrolyze the cleavable bond and obtain a short chained glycolipid and a hydroxy fatty acid.

7. The method of claim 6, **characterized in that** the alkaline hydrolysis is carried out in aqueous reaction medium at a pH of at least 8, preferably at least 13.

8. The method of claim 1, **characterized in that** the compound of formula I or its salt, methyl, ethyl or glycerol ester contains at least an unsaturated aliphatic bond and **in that** the glycolipid reaction product delivered by the micro-organism or its partially or fully deacetylated derivative is subjected to ozonolysis in the presence of ozone to break the unsaturated cleavable aliphatic bond.

9. The method of claim 8, **characterized in that** the ozonolysis is carried out at a pH of between 1 and 9, preferably between 4 and 8.

10. The method of any one of claims 1-9, **characterized in that** the reaction product of the hydrolysis reaction in alkaline medium and/or the reaction product of the ozonolysis is subjected to a hydrolysis in acidic medium at a pH of between 0.1 and 3, preferably between 0.1 and 1, with the purpose of cleaving the glycosidic bond.

11. The method of any one of claims 1-9, **characterized in that** the glycolipid is contacted with a glycosidase enzyme and subjected to an enzymatic de-glycosidation to obtain a glycosil unit and a hydroxy fatty acid, wherein the fatty acid is recovered.

12. The method of any one of claims 1-11, **characterized in that** the glycolipid contains a glycosil unit which is selected from the group of sophorose, trehalose, cellobiose, rhamnose or glucose.

13. The method of claim 12, **characterized in that** the glycolipid is a sophorolipid responding to the formula II 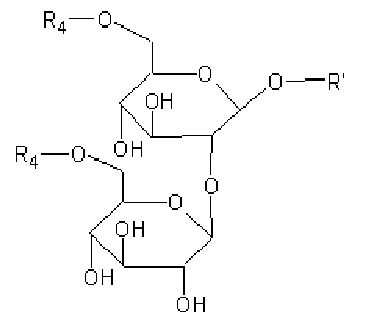 wherein
R⁴ is hydrogen or an acetyl group
R' is a -R¹⁰-R¹-X-Y-Z-R²-R²⁰ unit, wherein the 6-ring member is 2-O-β-D-Glucopyranosyl-α-D-glucose
wherein the sophorose group may be bound to R' on its ultimate or penultimate carbon atom.

14. The method of claim 12, **characterized in that** the glycolipid is a sophorolipid responding to the formula III, in which n and m may be the same or different and have a value of 0, 1 or 2.

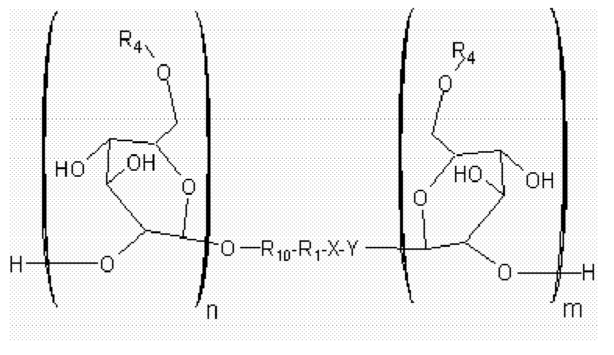

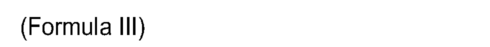

15. The method of any one of claims 1-14, **characterized in that** as the micro-organism used is a micro-organism selected from the group of *Candida apicola, Candida bogoriensis, Candida bombicola, Candida gropengiesseri, Candida magnoliae, Wickerhamiella domercqiae.*

16. An ω and/or ω-1 hydroxy fatty acid obtainable with the method according to any one of claims 10-14.

17. A sophorolipid which responds to the formula 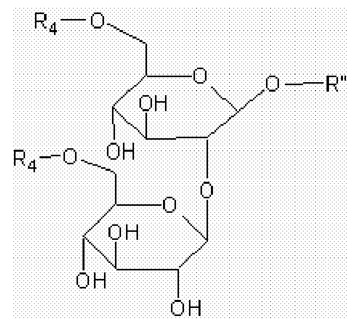 wherein
R⁴ is hydrogen or an acetyl group
R" is - R¹⁰-R¹-R³⁰ in which R³⁰ is any of the following groups -CH₃, -CHO, -CH₂OH, -COOH, -CHS, -CH₂SH, -CN or CH₂NR³ₚgroup, R³ being an aliphatic hydrocarbon chain or H, p being 1 or 2 wherein the sophorose group may be bound to R" on its ultimate or penultimate carbon atom.

18. Use of the glycolipid obtainable with the method of any one of claims 1-15 or the sophorolipid of claim 17, in a laundry washing composition, a dishwashing composition, a hard surface cleaning composition, in stay-on and rinse-off cosmetic applications.

19. Use of the reaction mixture obtainable with the method of any one of claims 6-15 as an antimicrobial agent.

20. A method for producing a hydroxy fatty acid, **characterized in that** the method comprises the step of subjecting a glycolipid obtained according to the method of any one of claims 1-15 to a hydrolysis reaction in acidic medium or to a de-glycosidation reaction in the presence of a glycosidase.
